(19) **Europäisches Patentamt / European Patent Office / Office européen des brevets**

(11) **EP 4 382 911 B1**

## (12) EUROPEAN PATENT SPECIFICATION

(45) Date of publication and mention
of the grant of the patent:
**28.01.2026 Bulletin 2026/05**

(21) Application number: **22383195.9**

(22) Date of filing: **07.12.2022**

(51) International Patent Classification (IPC):
**G01N 33/574** *(2006.01)*

(52) Cooperative Patent Classification (CPC):
**G01N 33/574**

(54) **COMBINING THE EXPRESSION LEVELS OF KRT19 AND COL1A2 TO PRODUCE A SCORE FOR SCREENING AND DIAGNOSING CANCER**

KOMBINATION DER EXPRESSIONSSTUFEN VON KRT19 UND COL1A2 ZUR ERZEUGUNG EINES SCORES FÜR DAS SCREENING UND DIE DIAGNOSE VON KREBS

COMBINAISON DES NIVEAUX D'EXPRESSION DE KRT19 ET COL1A2 POUR PRODUIRE UN SCORE POUR LE DEPISTAGE ET LE DIAGNOSTIC DU CANCER

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**12.06.2024 Bulletin 2024/24**

(73) Proprietor: **Oncomatryx Biopharma, S.L.**
**48160 Derio (ES)**

(72) Inventors:
- **Simón Buela, Laureano**
**48160 Derio (ES)**
- **Domínguez Hormaetxe, Saioa**
**48004 Bilbao (ES)**
- **Concha López, Ángel**
**15702 Santiago de Compostela (ES)**
- **Otero Alén, Maria**
**15702 Santiago de Compostela (ES)**
- **Otero Alen, Begoña**
**15220 Ames (ES)**
- **Estévez Pérez, Lara Sofía**
**15701 Santiago de Compostela (ES)**

(74) Representative: **Mewburn Ellis LLP**
**Aurora Building**
**Counterslip**
**Bristol BS1 6BX (GB)**

(56) References cited:
**WO-A1-2017/153546      US-A1- 2005 064 455**

- **CARÓN HELENA ET AL: "Identification of epigenetically regulated genes that predict patient outcome in neuroblastoma", BMC CANCER, BIOMED CENTRAL, LONDON, GB, vol. 11, no. 1, 11 February 2011 (2011-02-11), pages 66, XP021087277, ISSN: 1471-2407, DOI: 10.1186/1471-2407-11-66**
- **CSISZAR ANDREA ET AL: "Distinctive Molecular Composition of Human Gingival Interdental Papilla", JOURNAL OF PERIODONTOLOGY., vol. 78, no. 2, 1 February 2007 (2007-02-01), US, pages 304 - 314, XP093039063, ISSN: 0022-3492, DOI: 10.1902/jop.2007.060165**

Remarks:
The complete document including Reference Table(s) and the Sequence Listing(s) can be downloaded from the EPO website

## Description

### Field of the Invention

The present invention relates to methods for screening and diagnosing cancer, monitoring its clinical progression or response to treatment, and detecting recurrences, based on the combination of a tumor epithelial biomarker and a tumor stroma biomarker.

### Background of the Invention

Cancer is one of the main public health concerns worldwide. According to the GLOBOCAN database, administered by the International Agency for Research on Cancer belonging to the World Health Organization, almost 20 million cases of cancer were diagnosed worldwide in the year 2020, and the number of deaths due to cancer in the same year was near 10 million people.

The most common diagnostic procedure for diagnosing cancer when symptoms or other early markers are present are tissue biopsies. This involves taking a small sample of body tissue for analysis.

Alternatively, liquid biopsies can be carried out. These are performed on body fluids and are therefore less invasive than tissue biopsies. Currently, most liquid biopsy studies in cancer are being performed on circulating tumor cells (CTCs), circulating tumor DNA (ctDNA) and extracellular vesicles (EVs)[8] found in human plasma.

EVs are categorized in 2 classes: ectosomes and exosomes. Ectosomes [microvesicles (MVs) and oncosomes] measure 100 to 1000 nm and originate directly from plasma membrane budding[12]. Exosomes are vesicles formed in the endocytic pathway and range from 30 to 150 nm[12]. EVs can have different functions depending on the cell of origin; they have been intensively studied as facilitators of the immune response and also have a role in antigen presentation[11], programmed cell death, angiogenesis, inflammation, and coagulation[13,14]. Regarding cancer, EVs facilitate tumorigenesis and tumor progression, and are involved in the formation of premetastatic niche, tumor angiogenesis and tumor immune suppression[15,16].

In solid tumors, tumor-derived extracellular vesicle (TD-EV) trafficking has been observed[17]. The EVs carry complex, bioactive cargoes such as DNA, RNA, microRNA, and long non coding RNA (lncRNA)[18], in a mixture that depends on the microenvironment of the donor cell [14].

Biomarkers for prediction of cancer are known. For example, KRT19 is a known marker for the detection of dissemination of tumor cells in the breast, lung and colon cancer [19-21]. KRT19 is a type I keratin with low molecular weight. It is part of the cytoskeleton of numerous epithelial cells of different organs, and it is highly expressed in epithelial tumors. As such, it is a biomarker for detecting micro- and macrometastasis in several malignancies [20]. KRT19 has also been detected in exosomes derived from colon tumors by RT-PCR[22] and in circulating breast tumor cells [23].

The tumor microenvironment (TME) is formed of tumor stromal cells (stromal fibroblasts, endothelial cells and immune cells) and non-cellular components of extracellular matrix (ECM) such as collagen, fibronectin or laminin[24]. The non-malignant cells in the TME are key in tumor growth and progression, and are known to promote tumorigenesis in all phases of cancer development and metastasis[25]. ECM secreted by stromal cells also plays a crucial role in tumor progression, metastasis and therapeutic resistance[26]. Thus, TME components can reflect tumor status and therefore be useful as cancer diagnostic and prognostic biomarkers. Type I collagen (COL1A2) is a critical component of the ECM and its expression is increased in several types of cancer, such as pancreatic, colorectal, breast or lung[27-29]. Although COL1A2 gene expression has been used to characterize tumor progression *in vitro,* little has been researched about its clinical utility as a biomarker in human samples, besides a few studies relating to gastric and bladder cancers [30-34].

Although biomarkers for detection and diagnosis of cancer exist, many lack the sensitivity and specificity required for accurate diagnosis. Where biomarkers are found both in healthy tissue and malignant tissue, background noise can often drown out the signal from the malignant tissue.[42] Therefore there exists a need for cancer biomarkers with enhanced sensitivity.

US 2005/0064455 describes gene expression markers for predicting response to chemotherapy.

Carén et al. (2011, BMC Cancer) describes identification of epigenetically regulated genes that predict patient outcome in neuroblastoma.

Csiszar et al. (2007, J Peridontol) describes distinctive molecular composition of human gingival interdental papilla.

WO 2017/153546 describes a method for determining the risk of recurrence of an estrogen receptor-positive and HER2-negative primary mammary carcinoma under an endocrine therapy.

### Summary of the Invention

The present inventors have discovered that the combination of epithelial cell biomarkers and stromal cell biomarkers are useful for screening, detecting and monitoring cancers. Specifically, the combination of the epithelial tumor cell marker, KRT19, and tumor stromal cell marker, COL1A2, is useful for the discrimination of cancer patients from healthy donors. The combination of both biomarkers achieves much better sensitivity and specificity than each biomarker individually.

Accordingly, in a first aspect the invention provides a method for screening, detecting or monitoring a tumor in a subject, the method comprising:

 a). measuring the expression levels of KRT19 and COL1A2 in a sample obtained from the subject; and

 b). combining the expression levels of KRT19 and COL1A2 to produce a score

 c). based on the score, determining whether the subject has a high likelihood or low likelihood of a tumor.

In some embodiments, the subject is at risk of having or developing cancer. For example, the subject may have symptoms of cancer or have other risk factors.

In some embodiments, the subject is at risk of developing recurrence of cancer. For example, the subject may have symptoms of cancer or have other risk factors.

In some embodiments, the subject is a mammal. In preferred embodiments, the subject is a human.

In some embodiments, the sample obtained from the subject is a tissue sample.

In some embodiments, the sample obtained from the subject is a biological fluid (also **termed "liquid biopsy").** The biological fluid may be blood, serum, plasma, nipple aspirate fluid or urine. Such samples may be collected in less invasive ways than tumor tissue biopsies, which therefore makes the liquid biopsy approach more attractive for patients.

In some embodiments, the expression levels of KRT19 and COL1A2 are measured from plasma obtained from the subject.

Expression levels of KRT19 and COL1A2 may be measured from plasma as a whole and/or from any specific plasma derived component. Non limiting examples of plasma derived components include exosomes, ectosomes, extracellular vesicles, platelets, microvesicles, circulating tumor cells and circulating tumor DNA. Thus, in some embodiments, the expression levels of KRT19 and COL1A2 are measured from plasma derived exosomes, ectosomes, extracellular vesicles, platelets, microvesicles, circulating tumor cells and/or circulating tumor DNA obtained from the subject.

In some embodiments, the expression levels of KRT19 and COL1A2 are measured from plasma derived exosomes obtained from the subject.

In some embodiments, the tumor is cancerous and the cancer is a solid cancer. In some preferred embodiments, the cancer is selected from prostate cancer, pancreatic cancer, bladder cancer, renal cancer, melanoma, ovarian cancer, colorectal cancer, lung cancer, breast cancer, penile cancer and leiomyosarcoma.

In some embodiments, the method comprises measuring cDNA, RNA or protein expression levels of KRT19 and COL1A2.

In some embodiments, the expression levels of KRT19 and COL1A2 are measured in separate assays or a multiplexed assay.

In some embodiments, the RNA expression levels of KRT19 and COL1A2 are measured using RT-PCR.

In some embodiments, high expression levels of KRT19 and COL1A2 are positively correlated with a high likelihood of a tumor.

In some embodiments, combining the expression levels of KRT19 and COL1A2 to produce a score comprises using a logistic regression model.

In some embodiments, the score is a probabilistic score.

In some embodiments, combining the expression levels of KRT19 and COL1A2 to produce a score comprises:

 a). assigning a weight value to KRT19 expression to provide a weighted KRT29 expression level

 b). assigning a weight value to COL1A2 expression to provide a weighted COL1A2 expression level

 c). calculating the sum of the weighted KRT19 and COL1A2 expression levels

 d). using the sum obtained from step c) to produce a probabilistic score.

In some embodiments, combining the expression levels of KRT19 and COL1A2 to produce a probabilistic score comprises using the following formula:

$$probabilistic\ score\ p = \frac{1}{1 + e^{-(-\beta_0 + \sum_{i=1}^{k} \beta_i x_i)}}$$

wherein $\beta_0$ is an intercept weight, $\beta$ is a vector of weights for each of k variables and x is a vector of variables associated with the sample, wherein the variables comprise KRT19 expression levels, COL1A2 expression levels and optionally variables derived therefrom.

In some embodiments, the weights for the KRT19 expression level variable and the COL1A2 expression level variable have the same sign. In preferred embodiments the weights for the KRT19 expression level variable and the COL1A2 expression level variable both have positive coefficients.

In some embodiments determining based on the score, whether the subject has a high likelihood or low likelihood of a tumor comprises comparing said score with a reference value. In some embodiments the reference value is obtained from a control sample or is a pre-determined threshold.

In some embodiments determining based on the score, whether the subject has a high likelihood or low likelihood of a tumor comprises comparing said score with one or more predetermined thresholds, and determining that the subject has a high likelihood of a tumor if the score is above a first threshold, or determining that the subject has a low likelihood of a tumor if the score is below a second predetermined threshold, optionally wherein the first and second predetermined thresholds are the same.

When the method is used for monitoring a tumor, the score may be used to determine whether the tumor has progressed, regressed or stayed in a steady state compared to an earlier time point. Thus, in one embodiment, provided is a method for monitoring a tumor, the method comprising:

a). measuring the expression levels of KRT19 and COL1A2 in a sample obtained from the subject at a first time point;

b). combining the expression levels of KRT19 and COL1A2 obtained at the first time point to form a reference value;

c). measuring the expression levels of KRT19 and COL1A2 in a sample obtained from the subject at a second time point;

d). combining the expression levels of KRT19 and COL1A2 obtained at the second time point to produce a score;

e). based on the score, determining whether there is a high likelihood the tumor has progressed, regressed or stayed in a steady state when compared to the reference value.

In some embodiments the subject may be undergoing treatment for cancer. Thus, the method can monitor cancer progression or regression in a subject previously diagnosed with cancer in response to treatment.

In some embodiments, if it has been determined, based on the score, that there is a high likelihood of progression of the tumor, this suggests that the treatment or dosage of treatment should be revised.

In some embodiments, the treatment comprises chemotherapy, radiotherapy, treatment with biological agents such as antibodies, antibody conjugates, proteins or nucleotides, brachytherapy, or surgery.

In a second aspect, provided is a method comprising evaluating the efficacy of a treatment for cancer in a subject comprising:

a). measuring the expression levels of KRT19 and COL1A2 in a sample obtained from the subject at a first time point;

b). combining the expression levels of KRT19 and COL1A2 obtained at the first time point to form a reference value;

c). measuring the expression levels of KRT19 and COL1A2 in a sample obtained from the subject at a second time point;

d). combining the expression levels of KRT19 and COL1A2 obtained at the second time point to produce a score,

e). based on the score, determining whether there is a high likelihood the tumor has progressed, regressed or stayed in a steady state when compared to the reference value.

If the tumor has progressed or has stayed in a steady state, this indicates that the treatment is not efficacious. If the tumor has regressed, this indicates the treatment is efficacious.

In some embodiments, the treatment comprises chemotherapy, radiotherapy, treatment with biological agents such as antibodies, antibody conjugates, proteins or nucleotides, brachytherapy, or surgery.

The inventors have discovered that when KRT19 and COL1A2 RNA expression levels are measured from plasma derived exosomes from a subject, this provides a particularly powerful tool for predicting the likelihood of a tumor in a subject. Thus in some embodiments, provided herein is a method for screening, detecting or monitoring a tumor, the method comprising:

a). measuring the expression levels of KRT19 and COL1A2 in a plasma derived exosome sample obtained from the subject using RT-PCR;

b). combining the expression levels of KRT19 and COL1A2 comprising using the following algorithm:

$$probabilistic\ score\ p = \frac{1}{1 + e^{-(-1,396+0.550*KRT19+0.571*COL1A2)}}$$

c). based on the score, determining whether the subject has a high likelihood or low likelihood of a tumor.

In a third aspect provided is a method of providing a tool for characterizing a test sample obtained from a subject, the method comprising:

a). providing KRT19 and COL1A2 expression data for a plurality of training samples associated with known tumor status, thereby forming a labelled training dataset;

b). training a machine learning model using the labelled training dataset to learn parameters for at least the variable KRT19 expression level and the variable COL1A2 expression level, wherein the trained machine learning model provides as output a probabilistic score for predicting whether the test sample came from a subject having a tumor or not having a tumor, based on input data comprising the KRT19 expression level and COL1A2 expression level of the test sample.

The method of providing a tool for characterizing a test sample obtained from a subject described above may have any of the (computer-implemented) features described in relation to any embodiment of the first or second aspect of the invention.

In a fourth aspect provided is a computer implemented method for screening, detecting or monitoring a tumor in a subject, the method comprising:

a). providing KRT19 and COL1A2 expression data from a subject;

b). combining the expression levels of KRT19 and COL1A2 to produce a score;

d). based on the score, classifying the subject as having a high likelihood or low likelihood of a tumor. In some embodiments, the computer implemented method employs a machine learning model, which machine learning model has been trained using the labelled training dataset to learn parameters for at least the variable KRT19 expression level and the variable COL1A2 expression level, wherein the trained machine learning model provides as output a probabilistic score for predicting whether the test sample came from a subject having a tumour or not having a tumour, based on input data comprising the KRT19 expression level and COL1A2 expression level of the test sample.

In a fifth aspect, provided is a system comprising:

a). a processor; and

b). a computer readable medium comprising instructions that, when executed by the processor, cause the processor to perform the computer implemented method of the fourth aspect of the invention.

In a sixth aspect, provided is a non-transitory computer readable medium or media comprising instructions that, when executed by at least one processor, cause the at least one processor to perform the computer implemented method of the fourth aspect of the invention.

The computer implemented method of the fourth aspect of the invention, the system of the fifth aspect of the invention and the non-transitory computer readable medium or media of the sixth aspect of the invention may have any of the computer-implemented features described in relation to any embodiment of the first or second aspect of the invention.

In a seventh aspect, the invention provides use of a kit for detecting a tumor and/or for monitoring progression or regression of a tumor in a subject from a biological fluid sample obtained from the subject, the kit comprising reagents, and specific antibodies, primers or probes for detection and/or quantification of KRT19 and COL1A2 expression.

## Brief Description of the Drawings

Embodiments and experiments illustrating the principles of the invention will now be discussed with reference to the accompanying figures in which:

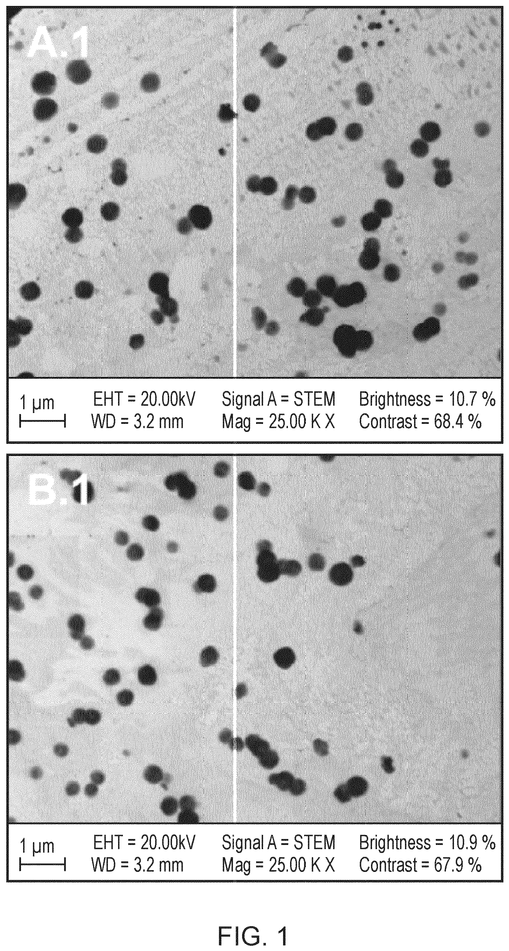

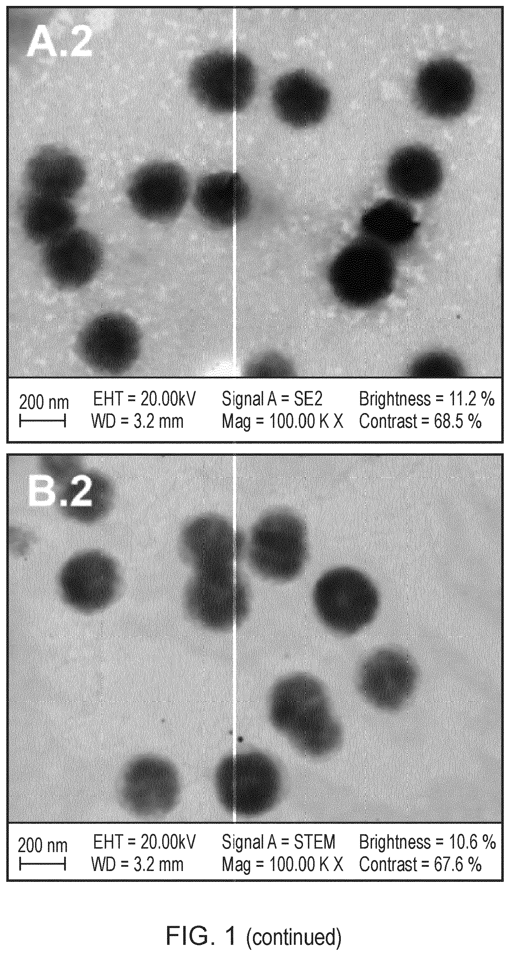

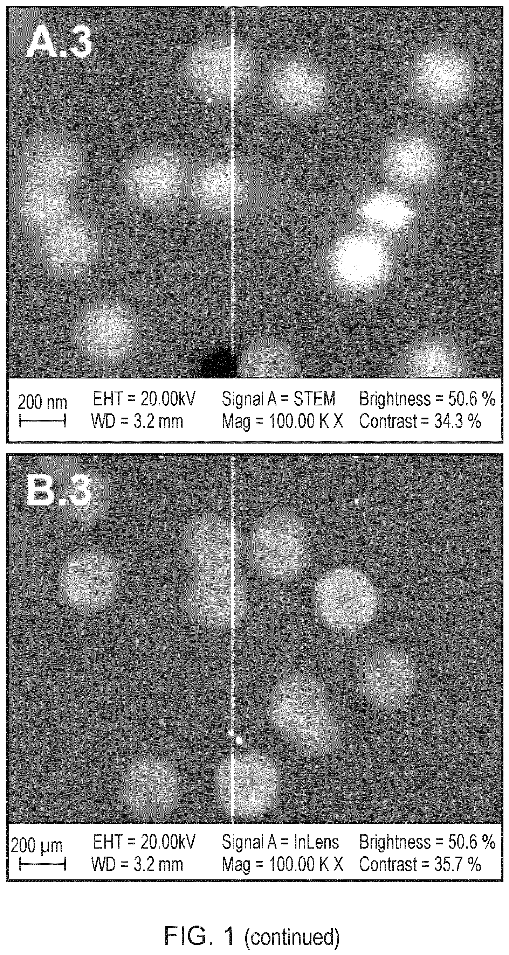

**Figure 1.** STEM micrographies of plasmatic extracellular vesicles. A. Extracellular vesicles from asymptomatic controls (AC). A1. TEM micrography of mixed microvesicles and **exosome structures at 25.00K of magnification. Scale bar 1 $\mu$m. A2. TEM micrography of** spheroid-shaped vesicles at 100.00K of magnification. Scale bar 200 nm. A3. SEM micrography of spheroid-shaped vesicles at 100.00K of magnification. Scale bar 200 nm. B. Plasmatic extracellular vesicles from cancer patients (CP). B1. TEM micrography of mixed **microvesicles and exosome structures at 25.00K of magnification. Scale bar 1 $\mu$m. B2. TEM** micrography of spheroid-shaped vesicles at 100.00K of magnification. Scale bar 200 nm. B3. SEM micrography of spheroid-shaped vesicles at 100.00K of magnification. Scale bar 200 nm. Images are representative of at least three independent experiments.

**Figure 2.** NTA of plasmatic extracellular vesicles. A. Concentration and size distribution of extracellular vesicles in plasma of AC (left) and CP (right) patients. B. Differences in concentration (B.1) and size distribution (B.2 and B.3) between AC and CP plasmatic samples. No differences were found between AC and CP plasmatic extracellular vesicles samples. The data were expressed as mean $\pm$ SD obtained from three independent experiments. Ns denotes no statistically significant differences when comparing CP with the control group.

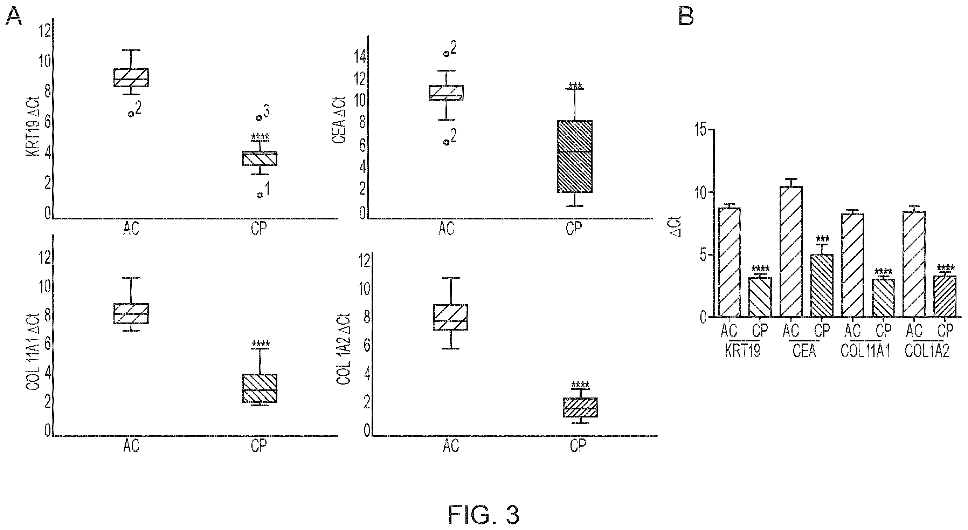

**Figure 3.** $\Delta$**Ct** values of KRT19, CEA, COL11A1 and COL1A2 in AC and CP **individuals. A. Box plot representation showing the distribution of $\Delta$Ct values of each gene. The median $\Delta$Ct value is represented as a black line within the box plot, and it divides the $\Delta$Ct** values into lower and upper quartile ranges. The whiskers represent the upper and lower data range in AC and CP samples. Data showed significant **differences in $\Delta$Ct values between two** cohorts of patients. **B. Comparison of $\Delta$Ct values of KRT19, CEA, COL11A1 and COL1A2 in** AC and PC samples. T tests showed significant difference in expression between the CP and AC groups for all biomarkers (KRT19, p-value<0.0001; CEA, p-value=0.0001; COL11A1, p-value<0.0001; COL1A2, p-value<0.0001). Data are expressed as mean $\pm$ SE. Asterisk denotes significant differences between groups AC and PC for each gene (**** p-value<0.0001; *** p-value<0.001; ** p-value<0.01; * p-value<0.05).

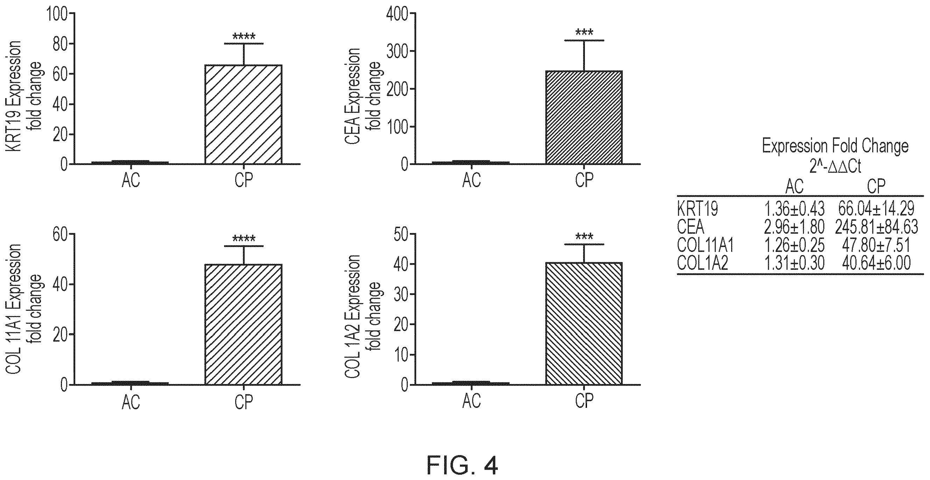

**Figure 4.** Graphs show relative fold expression of epithelial and stromal biomarkers (KRT19, CEA, COL11A1 and COL1A2) between AC and CP cohorts of patients calculated **using the 2-$\Delta\Delta$Ct method. Data showed higher expression levels of KRT19, CEA, COL11A1** and COL1A2 in CP individuals. Mann Whitney U test showed that these differences were significant between groups for all biomarkers (KRT19, p-value<0.0001; CEA, p-value=0.0003; COL11A1, p-value<0.0001; COL1A2, p-value=0.0001). Data are expressed as mean $\pm$ SE. Asterisk denotes significant differences between groups AC and PC for each gene (**** p-value<0.0001; *** p-value<0.001; ** p-value<0.01; * p-value<0.05).

**Figure 5.** ROC curves for KRT19, CEA, COL11A1 and COL1A2.

**Figure 6.** Amplification results of the individual reaction of ATCB. Different quantities of starting material were used to decipher the limit of detection for ACTB.

**Figure 7.** Amplification results of the individual reaction of KRT19. Different quantities of starting material were used to decipher the limit of detection for KRT19.

**Figure 8.** Amplification results of the individual reaction of COL11A1. Different quantities of starting material were used to decipher the limit of detection for COL11A1.

**Figure 9.** Amplification results of ACTB (purple), KRT19 (pink) and COL11A1 (blue) in the 3plex assay. Different quantities of starting material were used to decipher the limit of detection for all three of ACTB, KRT19 and COL11A1 in the same assay.

**Figure 10.** Amplification results of the individual reaction of COL1A2. Different quantities of starting material were used to decipher the limit of detection for COL1A2.

**Figure 11.** Amplification results of ACTB (purple), KRT19 (pink) and COL1A2 (green) in the 3plex assay. Different quantities of starting material were used to decipher the limit of detection for all three of ACTB, KRT19 and COL1A2 in the same assay.

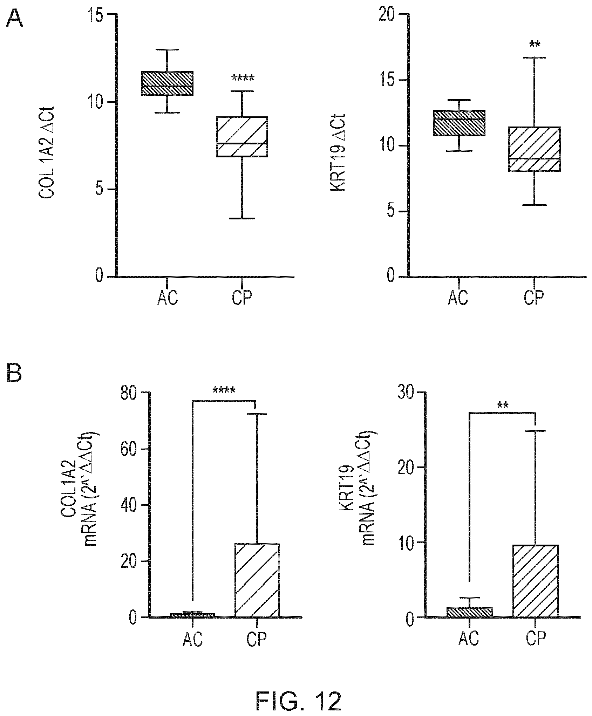

**Figure 12. Box plot showing the distribution of ΔCt values for tumor biomarkers** (COL1A2 and KRT19) in AC and CP. The y-**axis indicates ΔCt values and x**-axis indicates analyzed groups. Medians (black lines inside the boxes), interquartile ranges (width of the boxes representing the middle 50% of data) and total data ranges (whiskers) are shown. There was a significantly higher expression of the tumoral biomarkers in cancer patients **** p<0.0001 for COL1A2 and ** p = 0.0031 for KRT19. B The bar graph shows the mRNA fold change of COL1A2 and KRT19 of two independent groups AC and CP. LHS graph represents **mean ± SD of AC (1.40 ± 1.25) and CP (9.68 ± 15.07) for COL1A2 [AC (n = 12)** and CP (n = 30)]. RHS **graph represent mean ± SD of AC (1.21 ± 0.78) and CP (26.61 ±** 45.46) for KRT19 [AC (n = 13) and CP (n = 38)]. For significance analyses the Mann-Whitney U-test was used. COL1A2 and KRT19 showed significant difference, **** p<0.0001 and ** p = 0.0031 respectively.

**Figure 13.** COL1A2/KRT19 mRNA fold change in the different tumor types included in the study. Mann-Whitney U-test contrasts the mean in fold of control of two independent **groups, [AC vs cancer patients' groups (CRC (A and B), RC (C and D), BC (E and F), PC (G** and H). **Graphs represent mean ± SD, ****p < 0.0001, *** p < 0.001, ** p < 0.01, * p <0.05.**

**Figure 14.** ROC curves for A KRT19, B COL1A2, C KRT19 and COL1A2 combined. D shows ROC curves for A, B and C superimposed.

## Detailed Description of the Invention

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.
For the avoidance of any doubt, any theoretical explanations provided herein are provided for the purposes of improving the understanding of a reader. The inventors do not wish to be bound by any of these theoretical explanations.
Any section headings used herein are for organizational purposes only and are not to be construed as limiting the subject matter described.
Throughout this specification, including the claims which follow, unless the context requires **otherwise, the word "comprise" and "include", and variations such as "comprises", "comprising", and "including" will be understood to imply the inclusion of a stated integer or step or group of** integers or steps but not the exclusion of any other integer or step or group of integers or steps.
It must be noted that, as used in the specification and the appended claims, the singular forms **"a," "an," and "the" include plural referents unless the context clearly dictates otherwise. Ranges may be expressed herein as from "about" one particular value, and/or to "about" another** particular value. When such a range is expressed, another embodiment includes from the one particular value and/or to the other particular value. Similarly, when values are expressed as **approximations, by the use of the antecedent "about," it will be understood that the particular value forms another embodiment. The term "about" in relation to a numerical value is optional and** means for example +/- 10%.

### Definitions

To help understand the present patent application, the meanings of some of the terms and expressions as used in the context of the present invention are explained below.
The term "cancer" or "carcinoma" refers to the disease characterized by uncontrolled proliferation of abnormal cells capable of invading adjacent tissues and spreading to distant organs.
The term "specificity" refers to the capability of detecting true negatives. 100% specificity means that there are no false positives (subjects who do not have the disease but get a positive result).
The term "clinical progression" **or "progression"** generally refers to the advance of the clinical condition of the patient throughout the process of treating the disease. More specifically in the sense used in this description, the terms refer to a tumor that grows in size and/or progresses to further stages of the disease, reduces cell differentiation or increases invasiveness.

The **term "COL1A2"** refers to the gene collagen type I alpha 2 chain, ID 1278, also known as OI4; EDSCV; EDSARTH2, which codes for the protein COL1A2, also known as Collagen alpha-2(I) chain. Specifically, this gene encodes the pro-alpha2 chain of type I collagen whose triple helix comprises two alpha1 chains and one alpha2 chain. Type I is a fibril-forming collagen found in most connective tissues and is abundant in bone, cornea, dermis and tendon. Mutations in this gene are associated with osteogenesis imperfecta types I-IV, Ehlers-Danlos syndrome type VIIB, recessive Ehlers-Danlos syndrome Classical type, idiopathic osteoporosis, and atypical Marfan syndrome. Symptoms associated with mutations in this gene, however, tend to be less severe than mutations in the gene for the alpha1 chain of type I collagen (COL1A1) reflecting the different role of alpha2 chains in matrix integrity. Three transcripts, resulting from the use of alternate polyadenylation signals, have been identified for this gene.

The **term "exosome" refers to small, single**-membrane, secreted organelles of ~30 to ~200 nm in diameter that have the same topology as the cell and are enriched in selected proteins, lipids, nucleic acids, and glycoconjugates. Exosomes contain an array of membrane-associated, high-order oligomeric protein complexes, display pronounced molecular heterogeneity, and are created by budding at both plasma and endosome membranes. [35]

The term "gene" refers to a molecular chain of deoxyribonucleotides encoding a protein.

The term "KRT19" refers to the gene **"keratin 19", ID 3880,** encoding the protein cytokeratin type I cytoskeletal 19, also known as cytokeratin 19, keratin 19, K19 or CK19. The protein encoded by this gene is a member of the keratin family. The keratins are intermediate filament proteins responsible for the structural integrity of epithelial cells and are subdivided into cytokeratins and hair keratins. The type I cytokeratins consist of acidic proteins which are arranged in pairs of heterotypic keratin chains and are clustered in a region of chromosome 17q12-q21.

Unlike its related family members, this smallest known acidic cytokeratin is not paired with a basic cytokeratin in epithelial cells. It is specifically expressed in the periderm, the transiently superficial layer that envelopes the developing epidermis. It is expressed in a defined zone of basal keratinocytes in the deep outer root sheath of hair follicles and also observed in sweat gland and mammary gland ductal and secretory cells, bile ducts, gastrointestinal tract, bladder urothelium, oral epithelia, esophagus, ectocervical epithelium (at protein level). It is also expressed in epidermal basal cells, in nipple epidermis and a defined region of the hair follicle, and also seen in a subset of vascular wall cells in both the veins and artery of human umbilical cord, and in umbilical cord vascular smooth muscle and in muscle fibers accumulating in the costameres of myoplasm at the sarcolemma in structures that contain dystrophin and spectrin.

As it is used herein, the term "probability" **or"likelihood"** measures the frequency with which a result (or set of results) is obtained when performing a random experiment for which all the possible results are known, under sufficiently stable conditions. The probability or likelihood of obtaining a specific result can be "high", i.e., the frequency with which a result is obtained is greater than 50%, or "low", i.e., the frequency with which such result is obtained is less than 50%. As described herein, the probability that KTR19 and/or COL1A2 expression levels (e.g. RNA, cDNA or protein expression levels) are detected in a sample of a subject is higher when the subject has a tumor compared to in healthy subjects. Also, the probability of detecting higher levels of KRT19 and/or COL1A2 (e.g. RNA, cDNA or protein expression levels) is higher when the subject bears a tumor that is progressing rather than in remission. As a person skilled in the art will understand, probability does not have to be 100% for all the evaluated subjects, although it preferably should be. However, within the context of the present invention, said term requires that a statistically significant part of the subjects at risk of having a tumor for several reasons (risk factors, suspicious result in other tests, symptoms, or a previous tumor likely to recur), or of having a progressing tumor, can be identified as subjects having a higher probability of obtaining a specific result. The person skilled in the art can determine whether or not an event is statistically significant, without major complications, using different known statistical evaluation tools, for example, by means of **Student's** t-test, Mann-Whitney test, determining confidence intervals, determining the p-value, etc. Additional information about these statistical tools can be found in Dowdy and Wearden, Statistics for Research. John Wiley & Sons, New York 1983. Preferred confidence intervals are at least 50%, at least 60%, at least 70%, at least 80%, at least 90%, or at least 95%. P-values are preferably 0.1, 0.05, 0.02, 0.01 or less.

The term "recurrence" generally refers to the reoccurrence of the lesion after a relatively long period of absence of disease. The relatively long period includes more than 1 month, more than 2 months, more than 3 months, more than 4 months, more than 5 months, more than 6 months, more than 7 months, more than 8 months, more than 9 months, more than 10 months, more than 11 months, more than 1 year, more than 2 years, more than 5 years, or more than 10 years.

As it is used herein, the term "risk" refers to the relative risk or probability that a subject has a tumor in any location. A subject can be considered at risk based on different factors: clinically known risk factors (age, gender, family history of cancer, exposure to cancerous substances), symptoms, suspicious result in some tests (screening tests, image probes), or clinical history of past tumors with high rate of recurrence (bladder, colon, etc).

The term "sensitivity" refers to the detection of true positives (e.g., positive diagnosis of a pathology when the patient has said pathology); a 100% sensitivity means that there are no false negatives (negative diagnosis in patients that have said pathology).

The term "subject" or "individual" refers to a member of a mammalian species and includes, but is not limited to, domestic animals, primates and humans; the subject is preferably a male or female human being of any age or race.

The term "greater than" applied to RNA expression level, specifically KRT19 and COL1A2, means that the amount or concentration of said RNA in a specific sample of a subject with a tumor or a subject with a progressing tumor, is higher than in another sample considered as the control sample or reference value; therefore, the KRT19 and/or COL1A2 expression level in a sample from a subject with a tumor is higher (greater) than said KRT19 and/or COL2A1 RNA expression level in a control sample obtained from a control subject population without any history of cancer, or in previous samples from the same subject.

In the context of the present invention, it may be considered that the KRT19 and COL2A1 expression levels (e.g. RNA, cDNA or protein expression levels), in a sample from a subject with a tumor or with a progressing tumor, is greater than (or higher than) said expression levels in a control sample (reference value) when said expression level in the sample from the subject increases, for example, 3%, 5%, 10%, 25%, 50%, 100% or even more when compared with the reference value. In other embodiments it may increase, for example, at least 1.1-fold, 1.5-fold, 2-fold, 5-fold, 10-fold, 20-fold, 30-fold, 40-fold, 50-fold, 60-fold, 70-fold, 80-fold, 90-fold, 100-fold, or even more when compared to the reference value.

As it is used herein, the term "treatment" generally refers to the application of a therapy to alleviate or eliminate a pathology or to reduce or eliminate one or more symptoms associated with said pathology. Said therapy can include surgical intervention, pharmacological treatment, radiation therapy treatment, etc.

The term "tumor" refers to any abnormal mass of tissue resulting from a benign (noncancerous) or malignant (cancerous) neoplastic process.

**The term "RT-PCR multiplex assay"** refers to an assay wherein amplification of multiple specific nucleic acid sequences from RNA template(s) by PCR occurs within a single assay reaction using unique primers and/or probes for each specific sequence. The RT-PCR assay comprises purification, amplification, detection and discrimination steps.

**The term "purification"** in the context of exosomes means manual, semi-automated, and/or fully automated methods to purify tumor derived exosomes from a sample. In a preferred embodiment, the processing method utilizes the ExoRNeasy Maxi Kit, (QIAGEN, Hilden, Germany).

The term **"amplification" means generation of replicate copies of a specific** nucleic acid sequence present in the genome by providing target specific primer sequences within a PCR reaction that includes components needed for enzymatic replication, including dideoxynucleotides, magnesium, reverse transcriptase enzyme and amplicase enzyme in a suitable buffer. Amplification includes subjecting the reaction to repeated temperature cycles to allow for nucleic acid template denaturation, followed by primer annealing and extension from the primer sequences to generate replicate copies of the template.

**The term "detection"** in the context of the PCR assay means the method to measure amplification of a target sequence from a genetic template. Detection is achieved by the PCR instrument that is used for amplification. In one embodiment, the instrument used for detection is a real-time PCR detection system made by any manufacturer. In a more preferred embodiment, the instrument used for detection is the CFX96 C1000 Thermal Cycler (Bio-Rad Laboratories, USA).

**The term "sample" means human sample types including but not limited to** a tissue sample and fluid samples e.g. serum, blood, saliva, semen, perspiration, urine, tissue, synovial fluids, and/or tears. In **a preferred embodiment, the term "sample" means human** serum and/or blood. **The term "sample"** may be human serum.

**The term "reverse-transcription polymerase chain reaction" or "RT-PCR"** means executing real-time amplification in the presence of a fluorescently-labeled target-specific probe that binds the genetic template between the target-specific primers. The intact probe does not emit any fluorescent signal. Cleavage of the fluorescent dye-labeled probe during target amplification removes the presence of probe structural components or attached quencher dye that quenches the fluorescent dye signal on the intact probe. Upon probe cleavage, a detectable fluorescent signal is released which is measured by the PCR instrument. Result is reported as the PCR cycle where fluorescent dye is detectable above a threshold level. This PCR cycle can be read from a reference standard curve to generate a quantifiable template copy number in the original sample.

<u>Methods for the screening, detection and monitoring of tumors</u>

The present invention is based on the discovery that the combination of an epithelial cell marker and a stromal cell marker can be used as a sensitive biomarker for the screening, detection and monitoring of a tumor. Specifically, combining expression of the epithelial cell marker, KRT19, with the stromal cell marker, COL1A2, is a powerful tool for screening, detecting and monitoring a tumor in a subject. The biomarkers can be determined at protein, RNA or cDNA level, and may be present on tumor tissue, be free in plasma, or contained in plasma derived extracellular vesicles.

Accordingly, in a first aspect the invention provides a method for screening, detecting or monitoring a tumor in a subject, the method comprising:

a). measuring the expression levels of KRT19 and COL1A2 in a sample obtained from the subject; and

b). combining the expression levels of KRT19 and COL1A2 to produce a score

c). based on the score, determining whether the subject has a high likelihood or low likelihood of a tumor.

## Monitoring tumors

In spite of the advances in cancer treatment, drug resistance remains the main limiting factor for successful treatment of patients [40]. One of the strategies to overcome this problem is monitoring tumor progression during treatment, so treatment failure can be detected early, allowing a change of treatment or adaption to the current treatment as soon as possible.

The potential of tumor derived macrovesicles in treatment monitoring has been previously described in several types of cancer as breast, glioblastoma, colorectal, and even in non-solid cancers [41].

Accordingly, the invention provides a method for monitoring a tumor in a subject, the method comprising:

a). measuring the expression levels of KRT19 and COL1A2 in a sample obtained from the subject;

b). combining the expression levels of KRT19 and COL1A2 to produce a score;

c). based on the score, determining whether there is a high likelihood the tumor has progressed, regressed or stayed in a steady state when compared to the reference value.

When the method is used for monitoring cancer, the reference value may be a score obtained from the same subject at an earlier time point. When the method is used for monitoring cancer, the score obtained at a later time point may be indicative of progression, regression or steady state of the tumor when compared to the reference value. Steady state refers to a tumor that has not progressed or regressed. Thus, in one embodiment, provided is a method for monitoring a tumor, the method comprising:

a). measuring the expression levels of KRT19 and COL1A2 in a sample obtained from the subject at a first time point;

b). combining the expression levels of KRT19 and COL1A2 obtained at the first time point to form a reference value;

c). measuring the expression levels of KRT19 and COL1A2 in a sample obtained from the subject at a second time point;

d). combining the expression levels of KRT19 and COL1A2 obtained at the second time point to produce a score;

e). based on the score, determining whether there is a high likelihood the tumor has progressed, regressed or stayed in a steady state when compared to the reference value.

In one embodiment, when the method is used for monitoring a tumor, the subject may be undergoing treatment for cancer. Thus, the method can monitor cancer progression or regression in a subject previously diagnosed with cancer in response to treatment.

In some embodiments, if it has been determined, based on the score, that there is a high likelihood of progression of the tumor, this suggests that the treatment, schedule of administration or dosage of treatment should be revised. In some embodiments, if it has been determined, based on the score, that there is a high likelihood of progression of the tumor, this suggests that the treatment or dosage of treatment should be revised.

In some embodiments, the treatment comprises chemotherapy, radiotherapy, treatment with biological agents such as antibodies, antibody conjugates, proteins or nucleotides, brachytherapy, or surgery. A skilled person will realise that the subject may be undergoing any therapy used for treating solid cancers.

In some embodiments, the subject is not undergoing treatment for cancer. If the subject is not undergoing treatment, the methods of the invention may indicate that the subject needs to start treatment.

## Treatment efficacy

The combination of KRT19 and COL1A2 expression can also be used to determine the efficacy of a treatment by assessing tumor status in response to a particular treatment.

In a second aspect, provided herein is a method for evaluating the efficacy of a treatment for cancer in a subject comprising:

a). measuring the expression levels of KRT19 and COL1A2 in a sample obtained from the subject at a first time point;

b). combining the expression levels of KRT19 and COL1A2 obtained at the first time point to form a reference value;

c). measuring the expression levels of KRT19 and COL1A2 in a sample obtained from the subject at a second time point;

d). combining the expression levels of KRT19 and COL1A2 obtained at the second time point to produce a score,

e). based on the score, determining whether there is a high likelihood the tumor has progressed, regressed or stayed in a steady state when compared to the reference value.

If the tumor has progressed or has stayed in a steady state, this indicates that the treatment is not efficacious. If the tumor has regressed, this indicates the treatment is efficacious. In some embodiments, the treatment comprises chemotherapy, radiotherapy, treatment with biological agents such as antibodies, antibody conjugates, proteins or nucleotides, brachytherapy, or surgery. A skilled person would realise that the efficacy of any therapy used for treating solid cancers can be measured using the methods of the invention.

Subjects

The methods of the invention can be used to screen and detect tumors in subjects who have not previously been diagnosed with cancer. They can also be used for subjects who have previously been diagnosed with cancer, but have undergone a period of remission. In this way, the methods of the invention can detect recurrences of cancer.

In some embodiments, the subject is at risk of having or developing cancer. For example, the subject may have symptoms of cancer or have other risk factors. Risk factors include clinically known risk factors (age, gender, family history of cancer, exposure to cancerous substances), symptoms, suspicious result in some tests (screening tests, image probes), or clinical history of past tumors with high rate of recurrence (bladder, colon, etc).

In some embodiments, the subject is at risk of developing recurrence of cancer. For example, the subject may have symptoms of cancer or have other risk factors. Risk factors include clinically known risk factors (age, gender, family history of cancer, exposure to cancerous substances), symptoms, suspicious result in some tests (screening tests, image probes), or clinical history of past tumors with high rate of recurrence (bladder, colon, etc). The subject may have previously been diagnosed and treated for cancer and are in remission, for example the subject may have been in complete remission.

In some embodiments, the subject is a mammal. The subject may be any member of a mammalian species and includes, but is not limited to, domestic animals, primates and humans. In preferred embodiments, the subject is a human. The subject may be a male or female human being of any age or race.

In some embodiments, the tumour is cancerous. In some embodiments, the cancer is a solid cancer. Examples of solid cancers include AIDS related cancer, acoustic neoma, adenocystic carcinoma, adrenocortical cancer, agnogenic myeloid metaplasia, alopecia, alveolar soft-part sarcoma, anal cancer, angiosarcoma, aplastic anaemia, astrocytoma, ataxia-telangiectasia, basal cell carcinoma (bcc), bladder cancer, bone cancers, bowel cancer, brain stem glioma, brain and CNS cancers, breast cancer, CNS cancers, carcinoid cancers, cervical cancer, childhood brain cancers, childhood cancer, childhood soft tissue sarcoma, chondrosarcoma, choriocarcinoma, colorectal cancers, cutaneous T-Cell lymphoma, dermatofibrosarcoma-protuberans, desmoplastic small round cell cancer, ductal carcinoma, endocrine cancers, endometrial cancer, ependymoma, esophageal cancer, Ewing's sarcoma, extra hepatic bile duct cancer, eye cancer, eye: melanoma, retinoblastoma, fallopian tube cancer, fanconi anaemia, fibrosarcoma, gall bladder cancer, gastric cancer, gastrointestinal cancers, gastrointestinal carcinoid cancer, genitourinary cancers, germ cell cancers, gestational trophoblastic disease, glioma, gynecological cancers, hematological malignancies, head and neck cancer, hepatocellular cancer, hereditary breast cancer, histiocytosis, Hodgkin's disease, human papillomavirus, hydatidiform mole, hypercalcemia, hypopharynx cancer, intra-ocular melanoma, isle T-cell cancer, Kaposi's sarcoma, kidney cancer, Langerhan's cell histiocytosis, laryngeal cancer, leiomyosarcoma, li-fraumeni syndrome, lip cancer, liposarcoma, liver cancer, lung cancer, lymphedema, lymphoma, Hodgkin's lymphoma, non-Hodgkin's lymphoma, male breast cancer, malignant rhabdoid cancer of kidney, medulloblastoma, melanoma, merkel cell cancer, mesothelioma, metastatic cancer, mouth cancer, multiple endocrine neoplasia, mycosis fungoides, myelodysplastic syndromes, myeloma, myeloproliferative disorders, nasal cancer, nasopharyngeal cancer, nephroblastoma, neuroblastoma, neurofibromatosis, nijmegen breakage syndrome, non-melanoma skin cancer, non-small cell lung cancer (nsclc), ocular cancers, oesophageal cancer, oral cavity cancer, oropharynx cancer, osteosarcoma, ostomy ovarian cancer, pancreatic cancer, paranasal cancer, parathyroid cancer, parotid gland cancer, penile cancer, peripheral neuroectodermal cancers, pituitary cancer, polycythemia vera, prostate cancer, rare cancers and associated disorders, renal cancer, retinoblastoma, rhabdomyosarcoma, rothmund Thomson syndrome, salivary gland cancer, sarcoma, schwannoma, sezary syndrome, skin cancer, small cell lung cancer (sclc), small intestine cancer, soft tissue sarcoma, spinal cord cancers, squamous cell carcinoma (sec), stomach cancer, synovial sarcoma, testicular cancer, thymus cancer, thyroid cancer, transitional cell cancer (bladder), transitional cell cancer (renal-pelvis-/- ureter), trophoblastic cancer, urethral cancer, urinary system cancer, uroplakins, uterine sarcoma, uterus cancer, vaginal Cancer, vulva cancer, Waldenstrom's macroglobulinemia and Wilms' Cancer.

In preferred embodiments, the solid cancer is selected from bladder cancer, breast cancer, colorectal cancer, leiomyosarcoma, lung cancer, melanoma, ovarian cancer, pancreatic cancer, penile cancer, prostate cancer and renal cancer.

Samples

Expression of KRT19 and COL1A2 may be measured from a sample obtained from the subject.

In some embodiments, the sample obtained from the subject may be a tissue sample.

Tissue biopsies are still the main diagnostic procedure for diagnosis of cancer when symptoms or any other early markers are present. However, these types of biopsies are invasive, expensive, and harmful to the patients. Furthermore, they cannot be performed when the target lesion is difficult to access, or when the patients state of health is problematic[1]. Even if they allow a direct observation of the tissue, due to the heterogeneity and plasticity of neoplastic cells and their genetic variability, biopsies are not capable of reflecting the complete nature of the tumors, as they only picture a small part of the malignancy at a very specific moment [2]. Information that is crucial for a correct personalized patient management may therefore be lacking by using tissue biopsies [3]. After the first diagnosis, tissue biopsies are often still necessary for monitoring response after treatment in order to identify genetic alterations in the tumor, and therefore the same problems described above occur continually.

Therefore, it may be advantageous to carry out a liquid biopsy. A liquid biopsy is a non-invasive technique performed on body fluids that can be performed in any patient regardless their health state. The non-invasive nature of liquid biopsies allow repeated tests on the same patient, so they can be used for early detection/screening[5], detection of recurrences in asymptomatic patients, and monitoring response to treatment, while offering molecular information at different moments during disease evolution[6,7].

Thus, in some embodiments the sample obtained from the subject is a biological sample. In some embodiments, the sample obtained from the subject is a biological fluid. Illustrative, non-limiting examples of said sample include any biological fluid, such as blood, serum, nipple aspirate fluid, urine, etc. Depending on the type of samples, to simplify their storage and handling they can be frozen at -80°C after an initial sample processing.

In preferred embodiments, the sample obtained from the subject is a plasma sample. Plasma comprises many components. Examples of plasma components include exosomes, ectosomes, extracellular vesicles, platelets, microvesicles, circulating tumor cells and circulating tumor DNA. Expression levels of KRT19 and COL1A2 may be measured from the plasma sample as a whole and/or from any specific plasma derived component. The plasma derived component may be isolated from the plasma by any appropriate method. Non limiting examples of plasma derived components include exosomes, ectosomes, extracellular vesicles, platelets, microvesicles, circulating tumor cells and circulating tumor DNA. Thus, in some embodiments, the expression levels of KRT19 and COL1A2 are measured from plasma derived exosomes, ectosomes, extracellular vesicles, platelets, microvesicles, circulating tumor cells and/or circulating tumor DNA obtained from the subject.

In particular, plasma samples comprise circulating tumor cells (CTCs), circulating tumor DNA (ctDNA) and extracellular vesicles (EVs). Extracellular vesicles include exosomes and ectosomes. In some embodiments, the expression levels of KRT19 and COL1A2 are measured from CTCs, ctDNA or EVs derived from the plasma sample.

EVs show particular advantages over CTCs or ctDNA, as EVs are secreted by living cells, are found in large amounts in biofluids[9] and are more stable in circulating physiological conditions [8,10]. EVs contain unique molecular cargoes (nucleic acids, proteins or lipids) from donor cells, playing a crucial role in mediating intercellular communication[11].

In solid tumors, tumor-derived extracellular vesicle (TD-EV) trafficking has been observed[17]. They carry complex, bioactive cargoes such as DNA, RNA, microRNA, and long non coding RNA (lncRNA)[18], in a mixture that depends on the microenvironment of the donor cell [14]. This information transport allows communication among malignant cells in the tumor, but the TD-EVs may also travel to distant sites and modulate microenvironment there to form a premetastatic niche. Thus, epithelial and stromal EV-derived RNAs reflect tumor and tumor microenvironment status at every moment and can be detected by non-invasive procedures, making them great potential diagnostic, prognostic and predictive cancer biomarkers that could constitute a very useful tool in patients with active cancer disease [18].

In some embodiments the sample obtained from the subject is a plasma sample. Therefore, the expression levels of KRT19 and COL1A2 may be measured directly from the plasma. Alternatively or additionally, the expression levels of KRT19 and COL1A2 may be measured from plasma derived EVs obtained from the plasma sample. Alternatively or additionally, the expression levels of KRT19 and COL1A2 may be measured from plasma derived exosomes obtained from the plasma sample. In preferred embodiments, the expression levels of KRT19 and COL1A2 are measured from plasma derived exosomes obtained from the subject.

The plasma sample needs to be processed in order to purify the tumor-derived exosomes. One method for this purification is using the ExoRNeasy Maxi Kit, (QIAGEN, Hilden, Germany) according to the supplier's instructions.

Measurement of expression level

In some embodiments, the method comprises measuring cDNA, RNA or protein expression of KRT19 and COL1A2.

Expression can be detected by means of any conventional method which allows detecting the presence of a protein, cDNA or RNA. Illustrative, non-limiting examples of said methods which can detect protein include ELISA, immunoprecipitation, Western blotting and bead based multiplex immunofluorescent assays. Illustrative, non-limiting examples of said methods include which can detect RNA or cDNA include PCR, RT-PCR, multiplex PCR, nuclease protection assays and hybridization. A person skilled in the art will appreciate that any known technique for detecting the presence of a protein, cDNA or RNA may be used.

Determination of likelihood of tumor

Once the expression levels of KRT19 and COL1A2 have been measured, they are combined to produce a score. The score may be a probabilistic score.

High expression of KRT19 and COL1A2 are positively correlated with a high likelihood of a tumor. Low expression of KRT19 and COL1A2 indicate a low likelihood of a tumor.

Any suitable algorithm may be used to combine the KRT19 and COL1A2 expression levels. For example, combining the expression levels may comprise using a logistic regression model.

A weighted value may be assigned to each of KRT19 and COL1A2. The weighting may be based on the importance of the biomarker in the context of detecting tumors. A weight may be determined using an appropriate test dataset. The test data includes expression scores based on KRT19 and COL1A2 expression data from a plurality of samples with a known tumor status i.e. scores from samples from subjects with or without tumors.

In some embodiments, combining the expression levels of KRT19 and COL1A2 to produce a score comprises:

a). assigning a weight value to KRT19 expression to provide a weighted KRT29 expression level
b). assigning a weight value to COL1A2 expression to provide a weighted COL1A2 expression level
c). calculating the sum of the weighted KRT19 and COL1A2 expression levels
d). using the sum obtained from step c) to produce a probabilistic score.

In some embodiments, combining the expression levels of KRT19 and COL1A2 to produce a score comprises using the following formula:

$$probabilistic\ score\ p = \frac{1}{1 + e^{-(-\beta_0 + \sum_{i=1}^{k} \beta_i x_i)}}$$

wherein $\beta_0$ is an intercept weight, $\beta$ is a vector of weights for each of k variables and x is a vector of variables associated with the sample, wherein the variables comprise KRT19 expression levels, COL1A2 expression levels and optionally variables derived therefrom.

Variables derived therefrom include other parameters which may affect the likelihood of a subject having a tumor. Non-limiting examples include age of subject, sex of subject and cancer type.

In some embodiments, the weights for the KRT19 expression level variable and the COL1A2 expression level variable have the same sign. In preferred embodiments, the weights for the KRT19 expression level variable and the COL1A2 expression level variable both have positive coefficients.

The score produced by any of the methods described above determines whether there is a high likelihood or low likelihood of the subject having a tumor. In some cases, the score is indicative of the presence or absence of a tumor, preferably a tumor of any type.

Therefore in a subsequent step, the method involves correlating the score of combined data of KRT19 and COL1A2 with a high likelihood or low likelihood of a tumor. In some cases, the method involves correlating the score of combined data of KRT19 and COL1A2 with the presence or absence of a tumor.

Combination scores representing KRT19 and COL1A2 expression may be compared to a reference value. Scores representing expression levels of KRT19 and COL1A2 above the reference value may indicate the subject has a high likelihood of a tumor, while scores representing expression levels of KRT19 and COL1A2 below the reference value may indicate the subject has a low likelihood of a tumor. In some cases, scores representing expression levels of KRT19 and COL1A2 above the reference value indicate the subject has a tumor, while scores representing expression levels of KRT19 and COL1A2 below the reference value indicate the subject does not have a tumor.

The reference value may be obtained from a control sample. For example, the control sample may be from healthy individuals, in particular individuals with no medical history of cancer. When monitoring a tumor, the reference value may be obtained from the same subject at an earlier time point. When evaluating the efficacy of a treatment, the reference value may be obtained from the same subject at an earlier time point. The reference value may represent KRT19 and COL1A2 combined expression scores in the control sample (e.g. individuals with no medical history of cancer or samples from the same subjects at earlier time points).

The reference value may be a threshold value. For example, a pre-determined threshold value. There may be one pre-determined threshold value or there may be more than one pre-determined threshold values. For example, there may be two threshold values.

Thus, in some embodiments determining, based on the score, whether the subject has a high likelihood or low likelihood of a tumor comprises comparing said score with one or more predetermined thresholds, and determining that the subject has a high likelihood of a tumor if the score is above a first threshold, or determining that the subject has a low likelihood of a tumor if the score is below a second predetermined threshold, optionally wherein the first and second predetermined thresholds are the same.

The threshold values may be determined using test data. The test data includes expression scores based on KRT19 and COL1A2 expression data from a plurality of samples with a known tumor status i.e. scores from samples from subjects with or without tumors. The threshold values may be chosen to optimize accuracy of the tumor detection.

As described above, the score obtained from the methods of the invention determines whether there is a high likelihood or low likelihood of a subject having a tumor. This information can be used efficiently by a specialist for selecting the most suitable test in order to confirm the diagnosis and characterize the tumor, or the most suitable treatment to be administered to the subject diagnosed with cancer.

For methods involving monitoring tumor progression or regression, the combination score is compared with a previous value representing combined KRT19 and COL1A2 expression obtained from the same subject at an earlier time point. Comparison of the results indicates a high likelihood of progression, regression or steady state of the cancer.

Therefore in a subsequent step, this method involves correlating the increase or reduction in the KRT19 and COL1A2 combination score with the progression or regression of a tumor of any type and site.

This information can be used efficiently by a specialist for making a decision about initiating or ending a treatment, or modifying the current treatment doses or schedule of administration, or the drug itself.

For methods involving evaluating efficacy of a treatment, the combination score is compared with a previous value representing combined KRT19 and COL1A2 expression obtained from the same subject at an earlier time point. Comparison of the results indicates a high likelihood of progression, regression or steady state of the cancer.

Therefore in a subsequent step, this method involves correlating the increase or decrease in the KRT19 and COL1A2 combination score with the progression or regression of a tumor of any type and site. If the tumor has progressed, this indicates that the treatment is not efficacious. If the tumor has regressed, this indicates that the treatment is efficacious.

This information can be used efficiently by a specialist for making a decision about initiating or ending a treatment, or modifying the current treatment doses or schedule of administration, or the drug itself.

RT-PCR

In some embodiments, detection of KRT19 and COL2A1 is performed by means of an RT-PCR. This assay may be performed separately for each of the KRT19 and COL2A1 genes, or be multiplexed. In any case, values for each gene should be normalized using a housekeeping gene before analyzing the results. Any suitable house-keeping gene can be used. Non-limiting examples of house-keeping genes include beta-actin (ACTB), Glyceraldehyde 3-phosphate dehydrogenase (GAPDH), 18S rRNA and B2-microglobin ($\beta$2M).

Without being bound by any theory, it is thought that KRT19 and COL1A2 combination results will correlate with tumor size and progression, as bigger tumors will shred more epithelium and stroma derived material and tumor-derived exosomes, and thus result in KRT19 and COL1A2 combination expression levels being higher than in stable or regressing tumors.

Thus, provided herein is use of a kit as defined in the claims. An RT-PCR multiplex assay may be used for detecting and quantifying KRT19 and COL1A2 RNA levels in tumor derived exosomes from plasma, comprising:

a) an exosome purification step;

b) one or more amplification steps comprising real-time reverse-transcription polymerase chain reactions;

c) a detection step; and

d) a data combination step.

In some embodiments, the amplification step comprises a first reaction wherein KRT19 and COL1A2 RT-PCR **reactions are performed to determine how many copies of each gene's** RNA are present in the sample. KRT19 and COL1A2 RT-PCR reactions comprise amplifying nucleic acid using the KRT19 forward primer (SEQ ID NO: 1), KRT19 reverse primer (SEQ ID NO: 2), KRT19 probe (SEQ ID NO: 3), COL1A2 forward primer (SEQ ID NO: 4), COL1A2 forward primer (SEQ ID NO: 5), and COL1A2 probe (SEQ ID NO: 6). For data normalization purposes, expression of the ACTB gene is also determined simultaneously, using the ACTB forward primer (SEQ ID NO 7), ACTB reverse primer (SEQ ID NO 8) and ACTB probe (SEQ ID NO 9).

In some embodiments, the gene-specific primers and probes are fluorescently labeled. Preferred labels includes 5' hexachlorofluorescein (HEX),5' N-hydroxysuccinimide (LC610),5' tetramethylindo(di)-carbocyanines (CY5),or QSY quenchers or structure modifications such as 3' BlackBerry™ Quencher 650.

In another embodiment, the use of a kit as defined in the claims provides for alternative fluorescent dyes attached to the KRT19, COL1A2 and ACTB gene specific probes and multiplex dye combinations, as well as alternate quenchers or structural modifications to mask fluorescence while the probe is intact.

In some embodiments, provided is use of a kit as defined in the claims for detecting a tumor and/or monitoring progression or regression of a tumor in a subject from a biological fluid sample obtained from the subject, the kit comprising reagents, and specific antibodies, primers or probes for detection and/or quantification of KRT19 and COL1A2 expression. Any primers and probes which can detect and quantify KRT19 in a RT-PCR assay can be used. Any primers and probes which can detect COL1A2 in a RT-PCR assay can be used. The kit may comprise instructions for use.

In one embodiment the invention provides use of a kit for detecting KRT19 and COL1A2 in a sample as defined in the claims, the kit comprising the primers and probes selected from SEQ ID NOs: 1-9, and instructions for use.

Primers and probes which could be used to detect and quantify KRT19, COL1A2 and beta-actin are described below:

KRT19 RT-PCR:

Forward primer
5'-CAGCCACTACTACACGACCATC-3' (SEQ ID NO: 1);

Reverse primer
5'-CAAACTTGGTTCGGAAGTCATC-3' (SEQ ID NO: 2): and

Probe (TaqMan)
5'-CAGCCAGACGGGCATTGTCG-3' (SEQ ID NO: 3).

COL1A2-PCR:

Forward primer
5'-CCTGGACCAATGGGCTTA-3' (SEQ ID NO: 4);

Reverse primer
5'-GAAACCTTGAGGGCCTGG-3', (SEQ ID NO: 5); and

Probe (TaqMan)
5'-TAGAGGCCCACCTGGTGCAGC-3(SEQ ID NO: 6).

ACTS-PCR:

Forward primer

5'-CCACACTGTGCCCATCTACG -3' (SEQ ID NO: 7);

Reverse primer
5'-AGGATCTTCATGAGGTAGTCAGTCAG-3' (SEQ ID NO: 8); and

Probe (TaqMan)
5'-CAGGTCCAGACGCAGGATGGC-3'. (SEQ ID NO: 9).

Fluorophores:

5' hexachlorofluorescein (HEX)
5' N-hydroxysuccinimide (LC610)
5' tetramethylindo(di)-carbocyanines (CY5)

Quenchers:

3' BlackBerry™ Quencher 650 (BBQ-650™)

The cycle number (Ct) quantified for KRT19 and COL1A2 may be normalized using the Ct value obtained for the housekeeping gene (for example, ACTB). The normalized results may then be combined in order to produce a score. The score may be a probabilistic score.

In a particular embodiment, KRT19 and COL1A2 data are normalized using the $\Delta\Delta$Ct method. KRT19 and COL1A2 $\Delta\Delta$Ct values are then combined to produce a score. The score may be a probabilistic score.

High expression of KRT19 and COL1A2 are positively correlated with a high likelihood of a tumor. Low expression of KRT19 and COL1A2 indicate a low likelihood of a tumor.

Any suitable algorithm may be used to combine the KRT19 and COL1A2 expression levels. For example, combining the expression levels may comprise using a logistic regression model.

A weighted value may be assigned to each of KRT19 and COL1A2. The weighting may be based on the importance of the biomarker in the context of detecting tumors. A weight may be determined using an appropriate test dataset. The test data includes expression scores based on KRT19 and COL1A2 expression data from a plurality of samples with a known tumor status i.e. scores from samples from subjects with or without tumors.

In some embodiments, combining the expression levels of KRT19 and COL1A2 to produce as score comprises:

a). assigning a weight value to KRT19 expression to provide a weighted KRT29 expression level
b). assigning a weight value to COL1A2 expression to provide a weighted COL1A2 expression level
c). calculating the sum of the weighted KRT19 and COL1A2 expression levels
d). using the sum obtained from step c) to produce a probabilistic score.

In some embodiments, combining the expression levels of KRT19 and COL1A2 to produce a score comprises using the following formula:

$$probabilistic\ score\ p = \frac{1}{1 + e^{-(-\beta_0 + \sum_{i=1}^{k} \beta_i x_i)}}$$

wherein $\beta$o is an intercept weight, $\beta$ is a vector of weights for each of k variables and x is a vector of variables associated with the sample, wherein the variables comprise KRT19 expression levels, COL1A2 expression levels and optionally variables derived therefrom.

In some embodiments, the weights for the KRT19 expression level variable and the COL1A2 expression level variable have the same sign. In preferred embodiments, the weights for the KRT19 expression level variable and the COL1A2 expression level variable both have positive coefficients.

Variables derived therefrom include other parameters which may affect the likelihood of a subject having a tumor. Non-limiting examples include age of subject, sex of subject and cancer type.

In some embodiments, KRT19 and COL1A2 $\Delta\Delta$Ct values are combined using the following formula:

$$Probabilistic\ score\ p = \frac{1}{1 + e^{-(-1,396 + 0.550*KRT19 + 0.571*COL1A2)}}$$

This algorithm has been found to optimize the diagnostic power of KRT19 and COL1A2 combination expression level data obtained using an RT-PCR multiplex assay on RNA from plasma derived exosome samples from subjects. The assay consists of an exosome purification step followed by three real-time reverse-transcription polymerase chain reactions, detecting KRT19, COL1A2 and beta actin (ACTB) as normalizing gene. In one embodiment, exosomes are purified using ExoRNeasy Maxi Kit, (QIAGEN, Hilden, Germany). In a further embodiment, target amplification and detection are accomplished using TaqMan chemistry on the CFX96 C1000 Thermal Cycler (Bio-Rad Laboratories, USA).

After detection of the presence of KRT19 and COL1A2 RNA in a sample, quantification of the RNA copy number, normalization of the data and combination using the formula, it is possible to relate the final score of the test with a high likelihood or low likelihood of a tumor. Preferably, the score can relate to the presence or absence of a tumor of any type.

Therefore in a subsequent step, the method involves correlating a high score of combined data of KRT19 and COL1A2 with a high probablitty of a tumor, and the low score with a low probability of a tumor. In some embodiments, the high score of combined data of KRT19 and COL1A2 indicates presence of a tumor and low score indicates absence of a tumor.

Combination scores representing KRT19 and COL1A2 expression may be compared to a reference value. Scores representing expression levels of KRT19 and COL1A2 above the reference value may indicate the subject has a high likelihood of a tumor, while scores representing expression levels of KRT19 and COL1A2 below the reference value may indicate the subject has a low likelihood of a tumor. In some cases, scores representing expression levels of KRT19 and COL1A2 above the reference value indicate the subject has a tumor, while scores representing expression levels of KRT19 and COL1A2 below the reference value indicate the subject has no tumor.

The reference value may be obtained from a control sample. For example, healthy individuals, in particular individuals with no medical history of cancer. When monitoring a tumor, the reference value may be obtained from the same subject at an earlier time point. When evaluating the efficacy of a treatment, the reference value may be obtained from the same subject at an earlier time point. The reference value may represent KRT19 and COL1A2 combined expression scores in the control sample (e.g. individuals with no medical history of cancer or samples from the same subjects at earlier time points).

The reference value may be a threshold value. For example, a pre-determined threshold value. There may be one pre-determined threshold value or there may be more than one pre-determined threshold values. For example, there may be two threshold values.

Thus, in some embodiments determining, based on the score, whether the subject has a high likelihood or low likelihood of a tumor comprises comparing said score with one or more predetermined thresholds, and determining that the subject has a high likelihood of a tumor if the score is above a first threshold, or determining that the subject has a low likelihood of a tumor if the score is below a second predetermined threshold, optionally wherein the first and second predetermined thresholds are the same.

The threshold values may be determined using test data. The test data includes expression scores based on KRT19 and COL1A2 expression data from a plurality of samples with a known tumor status i.e. scores from samples from subjects with or without tumors. The threshold values may be chosen to optimize accuracy of the tumor detection.

As described above, the score obtained from the methods of the invention determines whether there is a high likelihood or low likelihood of a subject having a tumor. This information can be used efficiently by a specialist for selecting the most suitable test in order to confirm the diagnosis and characterize the tumor, or the most suitable treatment to be administered to the subject diagnosed with cancer.

In view of the foregoing, the person skilled in the art will comprehend that the invention further allows classifying subjects into healthy individuals or patients with active tumors, which is useful for deciding on the most suitable complementary test, treatment and/or follow-up regimen for each subject, depending on their situation. Therefore, the invention provides methods for the highly sensitive and specific *in vitro* detection of active tumors that may need to be treated or removed.

## Tools, computer implemented methods and systems

Determining whether a subject has a high or low likelihood of a tumor based on the KRT19 and COL1A2 combined expression score may comprise using a machine learning tool that has been trained using test data. The test data includes expression scores based on KRT19 and COL1A2 expression data from a plurality of samples with a known tumor status i.e. scores from samples from subjects with or without tumors.

Thus, also provided is a method of providing a tool for characterizing a sample obtained from a subject, the method comprising:

a). providing KRT19 and COL1A2 expression data for a plurality of training samples associated with known tumor status, thereby forming a labelled training dataset;

b). training a machine learning model using the labelled training dataset to learn parameters for at least the variable KRT19 expression level and the variable COL1A2 expression level, wherein the trained machine learning model provides as output a probabilistic score for predicting whether the test sample came from a subject having a tumor or not having a tumor, based on input data comprising the KRT19 expression level and COL1A2 expression level of the test sample.

The method of providing a tool for characterizing a test sample obtained from a subject described above may have any of the (computer-implemented) features described in relation to any embodiment of the first or second aspect of the invention.
The invention also provides a computer implemented method for screening, detecting or monitoring a tumor in a subject, the method comprising:

a). providing KRT19 and COL1A2 expression data from a subject;

b). combining the expression levels of KRT19 and COL1A2 to produce a score;

d). based on the score, classifying the subject as having a high likelihood or low likelihood of a tumor. In some embodiments, the computer implemented method employs a machine learning model to combine the expression levels of KRT19 and COL1A2 to produce a score, which machine learning model has been trained using the labelled training dataset to learn parameters for at least the variable KRT19 expression level and the variable COL1A2 expression level, wherein the trained machine learning model provides as output a probabilistic score for predicting whether the test sample came from a subject having a tumour or not having a tumour, based on input data comprising the KRT19 expression level and COL1A2 expression level of the test sample.

Also provided is a system comprising:

a). a processor; and

b). a computer readable medium comprising instructions that, when executed by the processor, cause the processor to perform the computer implemented method of the present invention.

The "system" may be a computer system and includes the hardware, software and data storage devices for embodying a system or carrying out a method as described above. For example, a computer system may comprise a processing unit such as a central processing unit (CPU) and/or graphics processing unit (GPU), input means, output means and data storage, which may be embodied as one or more connected computing devices. Preferably the computer system has a display or comprises a computing device that has a display to provide a visual output display. The data storage may comprise RAM, disk drives or other computer readable media. The computer system may include a plurality of computing devices connected by a network and able to communicate with each other over that network. It is explicitly envisaged that computer system may consist of or comprise a cloud computer. In certain embodiments the computer system may be operably connected to one or more devices for measuring gene expression of KRT19 and/or COL1A2. For example, the computer system may be operably connected to one or more Quantitative RT-PCR thermal cycler devices (such as a CFX96 C1000 Thermal Cycler) and able to receive data representing gene expression levels (such as ΔCt values for KRT19 and/or COL1A2) from the device.
Also provided is a non-transitory computer readable medium or media comprising instructions that, when executed by at least one processor, cause the at least one processor to perform the computer implemented method of the present invention.
As used herein, the term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.
The computer implemented method of the invention may have any of the features described in relation to any embodiment of the first or second aspect of the invention. As such, the system and non-transitory computer readable medium or media described above may also have any of the features described in relation to any embodiment of the first or second aspect. For example, the expression data provided may be obtained by any means described herein and from any subject described herein. Combining the expression level may be performed by means described herein and classifying, based on the score, the subject as having a high likelihood or low likelihood of a tumor may be achieved using any means described herein.

The following examples illustrate the invention and must not be considered in sense that limits said invention.

**EXAMPLES**

EXAMPLE 1

Purification and characterization of tumor derived exosomes from cancer patients

Materials and Methods

Peripheral whole blood was collected from each subject in a 10 mL EDTA-K2 tube and processed after centrifugation within 4 hours to avoid contamination with genomic DNA released from lysed blood cells. Samples were centrifuged at 2000 xg for 20 minutes to collect 2 to 4 mL of plasma. The plasma obtained was passed through a 0.8 $\mu$m filter and stored at -80 °C. Processing of plasma samples and RNA isolation were carried out using the commercial ExoRNeasy Maxi Kit, (QIAGEN, Hilden, Germany) and the manufacturer's protocols were followed. Total RNA was eluted in 14 $\mu$l of RNase-free water. Purified RNA from each sample was assayed qualitatively and quantitatively using the Agilent RNA 6000 Pico Kit on an Agilent 2100 Bioanalyzer.

In order to characterize their ultrastructural morphology, EVs were resuspended in 500 $\mu$l of XE buffer (QIAGEN, Hilden, Germany), samples were then adsorbed onto 300-mesh carbon-coated copper grids for 1 minute in a humidified chamber at room temperature. Grids with adhered EV were examined with a Zeiss Gemini SEM 500 microscope (Carl Zeiss Microscopy GmbH,Jena, Germany) equipped with a STEM detector at 20-30 kV.

The size distribution and concentration of plasmatic EVs was determined using Nanoarticle Tracking method (NTA), in a Malvern NanoSight NS300 Analyzer (Malvern Panalytical Ltd., Malvern, UK) with specific parameters according to the manufacturer's protocols. Captures and analysis were achieved using the built-in NanoSight Software NTA3.3.301 (Malvern Panalytical Ltd., Malvern, UK). The detection threshold for nanoparticles was fixed at 8 for all tests. Samples were diluted in PBS to a final volume of 1 mL. For each measurement, five consecutive 60 second videos were recorded at 25 ∘C, using a continuous syringe pump at an infusion speed of 40 (arbitrary units). Particles (EVs) were detected using a 488 nm laser (blue), and a scientific CMOS camera.

Results

To confirm the presence, morphology, and size of extracellular vesicles from the plasma of cancer patients (CP) and healthy individuals (AC), samples were observed by STEM microscopy technology. Vesicles ranging from 800 to 25 nm were analyzed. Micrographs showed mainly exosome and microvesicle structures in both samples (Figure 1). Morphology revealed a greater number of spheroid extracellular vesicles, with some cup-shaped exosome configurations (Figure 1A.1/1A.2). No differences were observed in structure or morphology between control donors and cancer patients.

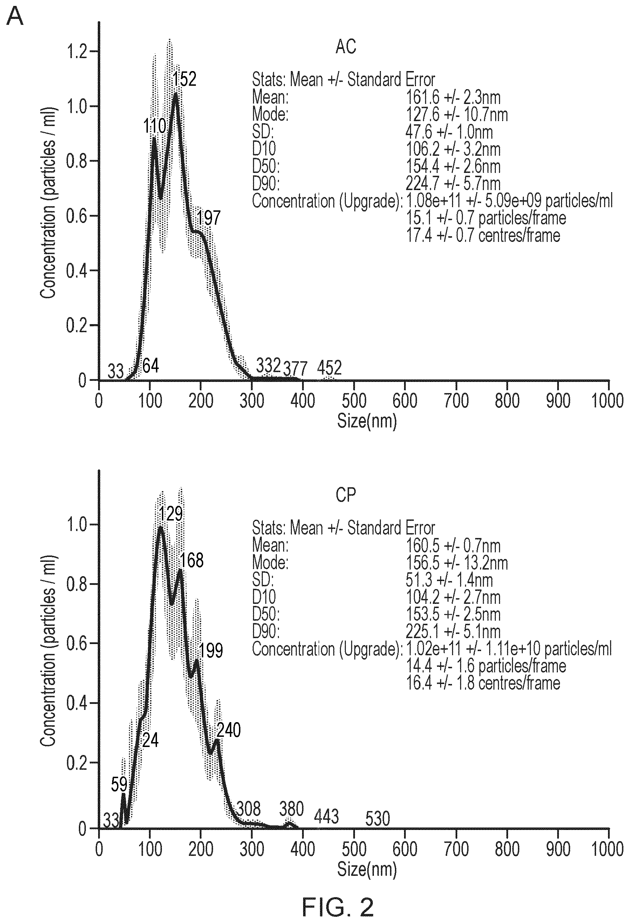

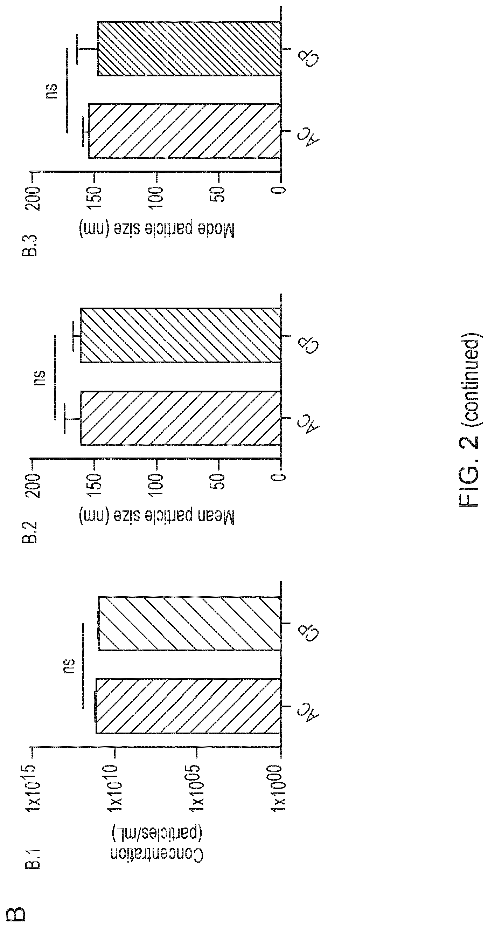

From NTA analysis, it was shown that the mean size of vesicles ranged from 161.6$\pm$2.3 nm (AC samples) to 160.5$\pm$0.7 nm (CP) (Figure 2). Mode values were similar (AC: 127.6$\pm$10.7nm; CP: 156.5$\pm$13.2nm). Analysis showed that exosome sized structures were mainly seen in both samples. Regarding concentration, AC samples showed a mean value of 1.08e$^{+11}$$\pm$5.09e$^{+9}$ particles/ml. Plasma of cancer donors registered a mean of 1.02e$^{+11}$$\pm$1.11e$^{+10}$ particles/ml. No statistically significant differences were found in concentration and size distribution between the two cohorts of donors.

Conclusions

The isolated EVs by STEM and NTA contained mainly exosome structures also with a considerable percentage of microvesicles. In concordance with other studies, no differences were found in concentration and size distribution between the two cohorts of donors.

EXAMPLE 2

Mono-plex analysis of RNA gene expression I

Materials and methods

Patients and Samples

A total of 30 plasma samples belonging to 30 patients were included in this study. This included ten healthy donors (AC) and 20 cancer patients (CP). All of them underwent blood extraction for healthcare causes and samples were analyzed in the Pathological Anatomy service at the University Hospital Complex of A Coruña. Demographic and clinical-pathological characteristics of the population are summarized in Table 1.

Regarding the control group corresponding to healthy patients, ten patients aged between 25 and 55 years who had not previously presented any oncological issues were analyzed. Patients also had no history of chronic pathologies, such as chronic inflammatory bowel disease (Crohn's disease, ulcerative colitis...), pancreatitis, cirrhosis, chronic obstructive pulmonary disease (COPD) or hypothyroidism, since it has been shown that this type of pathology may be related to alterations in the basal levels of KRT19[40,41] and CEA.[42-46] Among these 10 patients, 30% were men (n=3) and 70% were women (n=7).

In the group of cancer patients, plasma samples of eight prostate adenocarcinomas (seven staged IV and one staged IIb); three renal cell carcinomas (two staged IV, one staged III); three pancreatic neuroendocrine tumors (staged IV); two melanomas (both staged IV); one urothelial bladder carcinoma (stage IV); one patient with synchronous colorectal tumors, one adenocarcinoma and one small intestine neuroendocrine tumor (stage I and stage IV, respectively); one serous ovarian carcinoma (stage III), and one small-cell lung carcinoma (stage IV) were analyzed. Among these 20 patients, 85% were men (n=17) and 15% were women (n=3). The staging of the different tumors was evaluated according to the eighth edition of the TNM classification of the American Joint Committee on Cancer of 2017.[47]

Peripheral whole blood was collected from each subject in a 10 mL EDTA-K2 tube and processed after centrifugation within 4 hours to avoid contamination with genomic DNA released from lysed blood cells. Samples were centrifuged at 2000 xg for 20 minutes to collect 2 to 4 mL of plasma. The plasma obtained was passed through a 0.8 $\mu$m filter and stored at -80 °C until needed.

**Table 1**

| Clinical and pathological features of patients included in this study (n= 30) | | | |
|---|---|---|---|
| **Clinic pathological features** | **AC (n=10)** | | **CP (n=20)** |
| **Age median (range)** | 40.30 (25-55) | | |
| **Gender** | | | |
| Female | 7 (70.00%) | | 3 (15.00%) |
| Male | 3 (30.00%) | | 17 (85.00%) |
| **Clinical and pathological features of patients with tumoral process (n= 20)** | | | |
| **Prostate cancer (PC) (n=8)** | | **Renal cancer (PC) (n=3)** | |
| Age mean (range) | 73.25 (64-91) | Age mean (range) | 63.67 (63-65) |
| Histological Type | | Histological Type | |
| Adenocarcinoma | 8 | CCRC | 3 |
| Clinical stage | | Clinical stage | |
| I/II | 1 | I/II | 0 |
| III/IV | 7 | III/IV | 3 |
| **Pancreatic cancer (CRC) (n=3)** | | **Melanoma (CRC) (n=2)** | |
| Age mean (range) | 50.33 (41-65) | Age | 57-83 |
| Histological Type | | Histological Type | |
| Neuroendocrine carcinoma | 3 | Melanoma | 2 |
| Clinical stage | | Clinical stage | |
| I/II | 0 | I/II | 0 |
| III/IV | 3 | III/IV | 2 |
| **Others (n=4)** | | | |
| | Age | Histological Type | Clinical stage |
| Bladder carcinoma | 73 | Urotelial carcinoma | IV |
| Colorectal carcinoma | 63 | Adenocarcinoma/Neuroendocrine carcinoma | I/IV |
| Ovarian cancer | 70 | Serous adenocarcinoma | III |
| Lung cancer | 76 | Small-cell lung carcinoma (SCLC) | IV |

Measurement of RNA

RNA was quantified using the Agilent 2100 Bioanalyzer, and total RNA samples were reverse transcribed into cDNA according to the Quantinova Reverse Transcription kit (QIAGEN, Hilden, Germany) protocol. The synthesis of cDNA was carried out using a thermal cycler **following manufacturer's instructions: 2 min at 45°C for gDNA elimination reaction, 3 min at 25°C** for annealing step, 10 min at 45 °C for reverse transcription step and 5 min 85 °C to inactivate the reverse transcriptase.

Quantitative RT-PCR was performed in a CFX96 C1000 Thermal Cycler (Bio-Rad Laboratories, USA). cDNA expression was assessed using QuantiNova SYBR Green PCR kit (QIAGEN, Hilden, Germany). PCR conditions were set according to the supplier's instructions. Briefly: initial activation at 95°C for 2 min and 45 cycles of 95°C for 5 s, 60°C for 10 s. Analysis of **the melting curves: increase of 0.5° C from 55° C to 95 °C. PCRs were performed in 20 $\mu$L reaction volumes, containing 6 $\mu$L H2O, 10 $\mu$L QuantiNova SYBR Green PCR, 1 $\mu$L of each primer** [20 $\mu$M forward/reverse primers (TIB Molbiol, Berlin, Germany), final concentration: 1 $\mu$M], and 2 $\mu$L **cDNA template. Forward and reverse primers are listed in Table 2.**

**Table 2. Primers used in qRT-PCR**

| PRIMER NAME | FORWARD PRIMERS | REVERSE PRIMERS |
|---|---|---|
| ACTB (Housekeeping) | 5'AGCCTCGCCTTTGCCGA 3'(SEQ ID NO:10) | 5'CTGGTGCCTGGGGCG 3'(SEQ ID NO:11) |
| KRT19 | 5'CAGCCACTACTACACGACCATC 3' (SEQ ID NO: 1) | 5'CAAACTTGGTTCGGAAGTCATC 3'(SEQ ID NO: 2) |
| CEA | 5'AATGGGATACCGCAGCAAC 3'(SEQ ID NO:12) | 5'GAGAGACCAGGAGAAGTTCCAGAT 3' (SEQ ID NO:13) |
| COL11A1 | 5'AATGGAGCTGATGGACCACA 3' (SEQ ID NO:14) | 5'TCCTTTGGGACCGCCTAC 3'(SEQ ID NO:15) |
| COL1A2 | 5'TCAAACTGGCTGCCAGCAT 3' (SEQ ID NO:16) | 5'CAAGAAACACGTCTGGCTAGG 3' (SEQ ID NO:17) |

Relative gene expression was calculated using a modified comparative threshold cycle (Ct) method, (2-$\Delta\Delta$Ct), as described previously by Pfaffl.[39] Two replicates of each sample were analyzed for each gene. For the housekeeping gene (ACTB), a Ct$\leq$28 was considered positive. ACTB Ct values $\geq$29 were considered negative and results were considered invalid. For epithelial and stromal markers, a Ct value $\leq$39 with a sigmoidal curve was accepted as positive. After amplification, a representative sample from each set of amplicons was analyzed by agarose electrophoresis to confirm their specificity. 16 microliters of PCR products were separated by electrophoresis on a 2% agarose gel.

Statistical analysis

Statistical analyses were performed using the IBM SPSS® Statistics v27 program. Descriptive statistics were used for characterizing the clinical and pathological data of the patients in the study. A Shapiro-Wilk normality test was performed for each data set. The Grubbs and Dixon test was carried out to detect atypical data (outliers). Box plot diagrams were performed for study of the distribution of $\Delta$Ct values between AC and PC groups. T-tests were performed for comparisons between the two groups when the statistical data followed a normal distribution. The nonparametric Mann-Whitney U test was used for comparisons of relative fold expression among the two cohorts of patients. $\Delta$Ct and 2-$\Delta\Delta$Ct values were represented as mean$\pm$SEM. Statistical significance was determined at $\alpha$-limit = 5%

Results

$\Delta$Ct values obtained for each sample in the AC and CP cohorts are detailed in **Tables 3(I) and (II),** respectively. $\Delta$Ct values were obtained for two epithelial markers and two stromal markers. The two epithelial markers were KRT19 and CEA. The two stromal markers were COL11A1 and COL1A2. All data were normalized to ACTB expression. $\Delta$Ct indicates the difference between the mean of two replicates of the Ct of the target gene and the mean of the Ct of the reference control gene (ACTB).

**Table 3 (I)**

| Patient | Sex | RNA Expression | | | |
|---|---|---|---|---|---|
| | | ΔCt KRT19 | ΔCt CEA | ΔCt COL11A1 | ΔCt COL1A2 |
| AC1 | Female | 8,27 | 8,16 | 10,59 | 9,25 |
| AC2 | Female | 8,58 | 6,12 | 9,5 | 7,36 |
| AC3 | Female | 6,31 | 14,21 | 8,27 | 8,85 |
| AC4 | Male | 7,68 | 10,32 | 6,76 | 9,35 |
| AC5 | Female | 8,67 | 10,56 | 7,54 | 10,81 |
| AC6 | Male | 9,35 | 9,95 | 8,67 | 7,73 |
| AC7 | Male | 9,32 | 11,25 | 7,36 | 6,64 |
| Ac8 | Female | 8,65 | 10,32 | 8,45 | 8,27 |
| AC9 | Female | 10,65 | 9,99 | 7,25 | 7,79 |
| AC10 | Female | 8,95 | 12,65 | 7,68 | 8,32 |
| | Mean | 8,64 | 10,35 | 8,21 | 8,44 |
| | SEM | 0,36 | 0,70 | 0,37 | 0,37 |

**Table 3 (II)**

| Patient | Stage | Sex | RNA Expression | | | |
|---|---|---|---|---|---|---|
| | | | ΔCt KRT19 | ΔCt CEA | ΔCt COL11A1 | ΔCt COL1A2 |
| Prostate cancer | IV | Male | 0,88 | 5,41 | 1,33 | 2,59 |
| Prostate cancer | IV | Male | 3,84 | 6,7 | 2,51 | 2,87 |
| Prostate cancer | IV | Male | 6,09 | 10,97 | 3,66 | 4,2 |
| Prostate cancer | IV | Male | 2,29 | 0,68 | 2,28 | |
| Prostate cancer | IV | Male | 3,61 | 3,92 | 5,42 | |
| Prostate cancer | IIB | Male | 3,71 | 1,57 | 1,62 | 2,15 |
| Prostate cancer | IV | Male | 2,94 | 5,24 | 1,32 | |
| Prostate cancer | IV | Male | 4,55 | 8,87 | 3,55 | |
| Renal cancer | IV | Male | 3,81 | 8,07 | 2,81 | 3,45 |
| Renal cancer | IV | Male | 2,97 | 0,28 | 2,11 | 2,52 |
| Renal cancer | III | Male | 0,73 | 0,28 | 4,98 | 4,06 |
| Pancreatic NET | IV | Male | 3,15 | 6,84 | 2,11 | |
| Pancreatic NET | IV | Male | 3,56 | 8,58 | 3,96 | |
| Pancreatic NET | IV | Male | 3,31 | 7,54 | 2,07 | |
| Melanoma | IV | Male | 1,51 | 6 | 2,99 | 3,25 |
| Melanoma | IV | Female | 3,02 | 9,42 | 4,58 | 2,78 |
| Bladder carcinoma | IV | Female | 3,18 | 1,93 | 2,98 | 3,61 |
| Colorectal carcinoma+NET | IV | Male | 5,34 | 4,61 | 3,65 | 4,44 |
| Ovarian cancer | III | Female | 2,1 | 3,53 | 3,25 | |
| Lung cancer | IV | Male | 2,29 | 0,73 | 3,1 | |
| | | Mean | 3,14 | 5,06 | 3,01 | 3,27 |
| | | SEM | 0,30 | 0,75 | 0,26 | 0,23 |

Box plot diagrams to visualize the distribution of the ΔCt results in AC and CP cohorts were created and comparisons between both groups were made **(Figure 3).** Distribution of the data showed that the median ΔCt for KRT19 in AC and CP was 8.66 (IQR: 1.21) and 3.66 (IQR: 1.24), respectively. Median ΔCt for CEA was 10.32 (IQR: 2.10; AC) and 5.32 (IQR: 6.92; PC). This last result indicated a great dispersion and variability of the data of CP group for CEA. Stromal markers presented a median ΔCt for COL11A1 of 7.97 (IQR: 1.54; AC) and 2.39 (IQR: 2.03; PC) and a median ΔCt for COL1A2 of 8.29 (IQR: 1.64; AC) and 3.25 (IQR: 1.47; PC) (Figure 3A). Box plot diagrams also showed the presence of extreme data in ΔCt values in KRT19 (AC and CP samples), and in CEA AC results. Grubbs and Dixon test analysis of the outliers were performed for these values and confirmed that these data were furthest from the rest but were not significant outliers (p-value>0.05; IC:95%).

Figures 3A and B also show that differences in ΔCt values between AC and CP groups were significantly different in all genes studied. T tests yielded a p-value<0.0001 for KRT19, p-value=0.0001 for CEA, p-value<0.0001 for COL11A1 and a p-value<0.0001 for COL1A2.

Relative gene expression for each biomarker was evaluated using a modified comparative Ct method, (2-ΔΔCt), as described previously by Pfaffl[39]. Figure 4 shows fold change in expression of AC and CP samples. The results showed increased expression in KRT19 for CP patients (66.04 ± 14.29) in comparison to AC patients (1.36 ± 0.43). CEA levels were also highly incremented in cancer individuals (245.81 ± 84.63) relative to healthy donors (2.96 ± 1.80). However, a high interindividual variability in expression was found for this gene in both the AC and CP cohort, even within oncological patients with the same type of cancer. Stromal markers were also highly elevated in the CP group. AC patients showed values of 1.26 ± 0.25 (COL11A1) and 1.31 ± 0.30 (COL1A2), whereas expression in CP patients for COL11A1 and COL1A2 increased 47.80 ± 7.51-fold and 40.64 ± 6.00-fold, respectively. Both groups were found to be significantly different using a non-parametric test for all epithelial and stromal biomarkers. Mann Whitney U tests yielded a p-value <0.0001 for KRT19, p-value=0.0003 for CEA, p-value<0.0001 for COL11A1 and p-value=0.0001 for COL1A2.

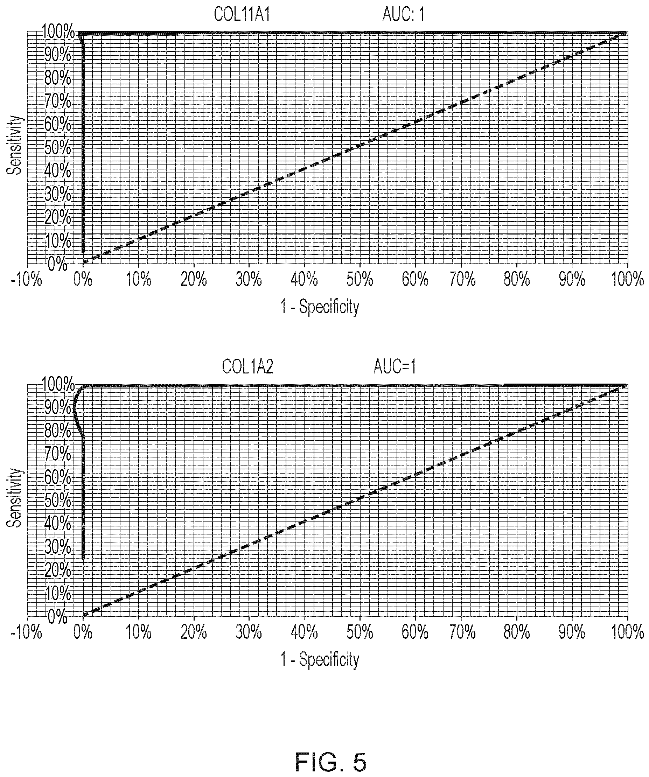

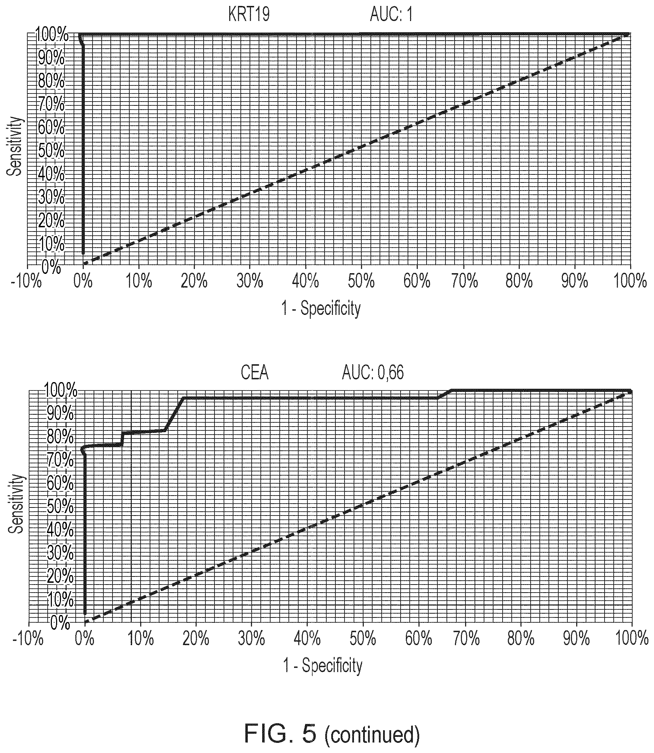

ROC curves were performed to evaluate the diagnostic potential of the epithelial and stromal markers, as a basis to decide which of them include in the final combination (**Figure 5**).

KRT19 yielded an AUC of 1 (reaching sensitivity and specificity values of 100%) while CEA yielded an AUC of 0,66 (with sensitivity and specificity of 95-83%), thus, KRT19 was selected as the epithelial biomarker to build the final combination. For the stromal markers, both COL11A1 and COL2A1 obtained AUC values of 1, with sensitivity and specificity values of 100%.

Conclusions

KRT19, CEA, COL11A1 and COL1A2 showed significant differences in expression between control and cancer groups, validating their potential as cancer biomarkers in plasma exosomes from patients with different types of tumors of advanced stages (III/IV), including prostate, renal, pancreatic, melanoma, ovary, bladder, colorectal and lung cancers. However, with regards to epithelial markers, KRT19 displayed higher sensitivity and specificity values than CEA.

EXAMPLE 3

Development of multiplex assay

Materials and methods

For the development of the multiplex assay, Qiagen's Quantinova Multiplex RTPCR system was selected, and reactions were performed in a CFX96 C1000 Thermal Cycler (Bio-Rad Laboratories, EEUU).

The primers described herein for ACTB, KRT19, COL11A1 and COL1A2 were used, along with the following TaqMan probes:

ACTB_P660 Length: 21 mer
Cy5-CAggTCCAgACgCAggATggC-BBQ (SEQ ID NO: 9)

KRT19_P610 Length: 20 mer
LC610-CAgCCAgACgggCATTgTCg-BBQ (SEQ ID NO: 3)

COL11A1_P530 Length: 25 mer
6FAM-CTCCAACACCACCAACTgAACCAAC-BBQ (SEQ ID NO.18)

COL1A2_P580 Length: 21 mer
HEX-TAgAggCCCACCTggTgCAgC-BBQ (SEQ ID NO: 6)

Results

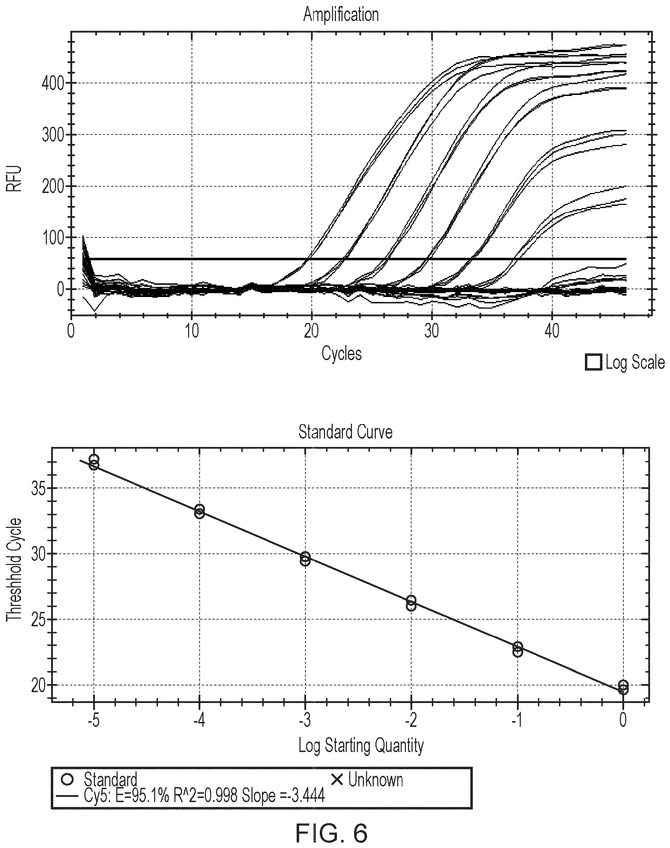

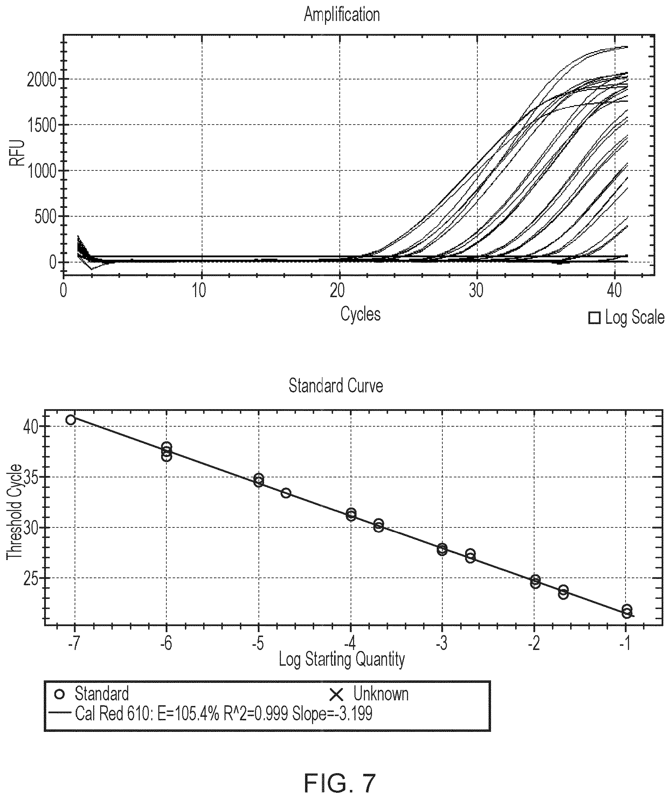

First, individual reactions were optimized. Both ACTB and KRT19 reactions performed well, with detection limits of 2 and 0.2 pg RNA respectively (**Figures 6 and 7**).

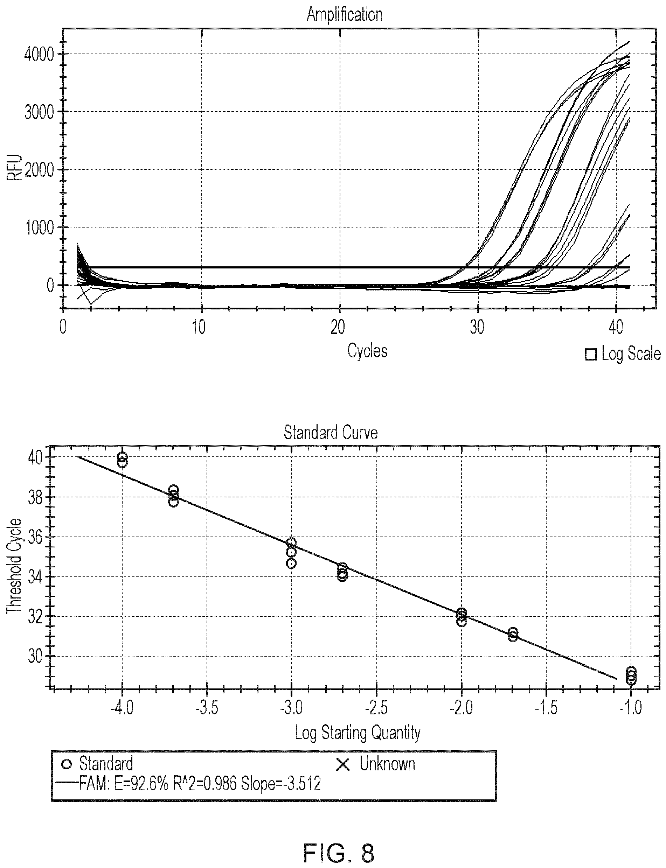

However, the COL11A1 was found to be much less sensitive, with a detection limit of 200 pg RNA (**Figure 8**).

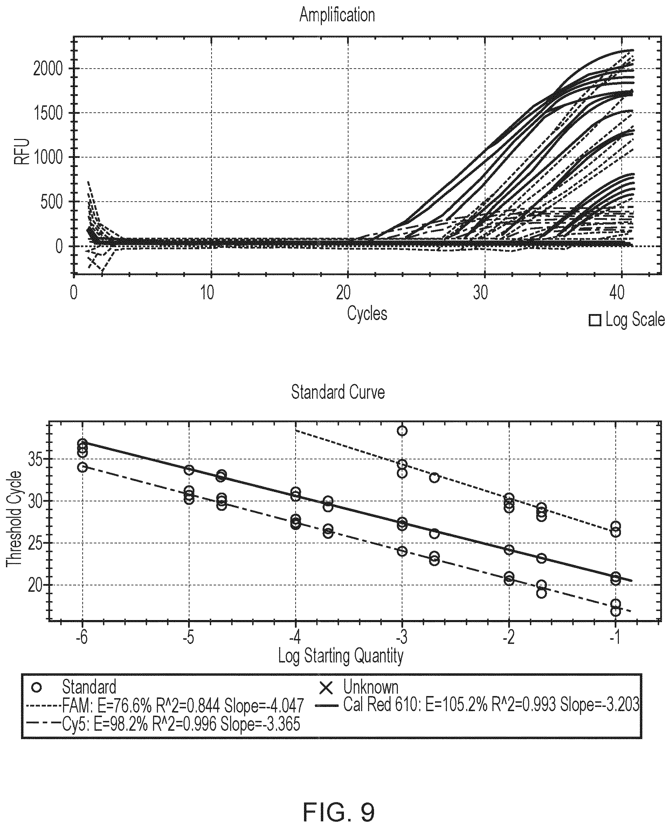

When all three reactions were multiplexed, ACTB and KRT19 reactions maintained their performance, but for COL11A1, the sensitivity was reduced even more (**Figure 9**).

After several unsuccessful attempts to improve COL11A1 detection, even testing different primers, COL11A1 was discarded and COL1A2 was finally selected as the stromal marker for the multiplexed combination.

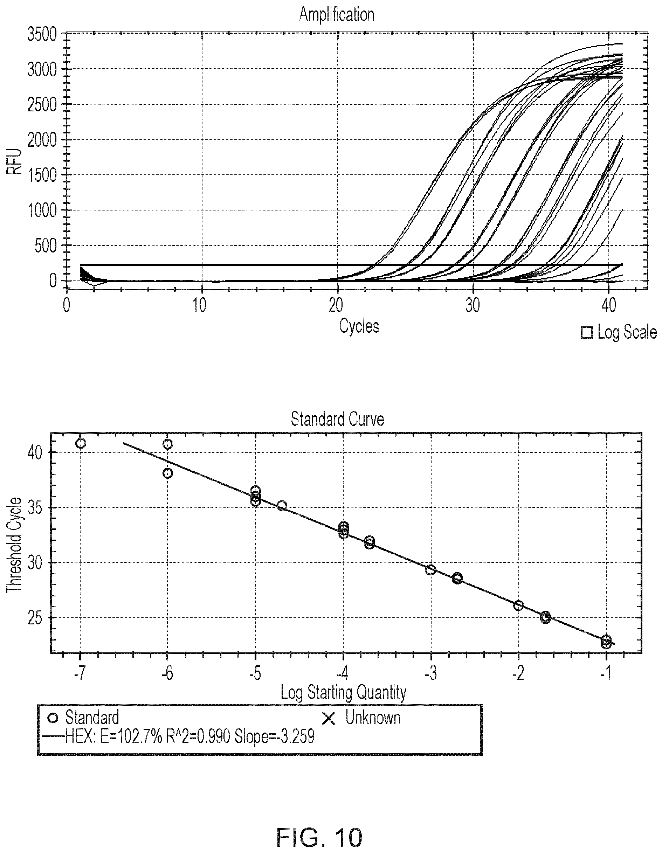

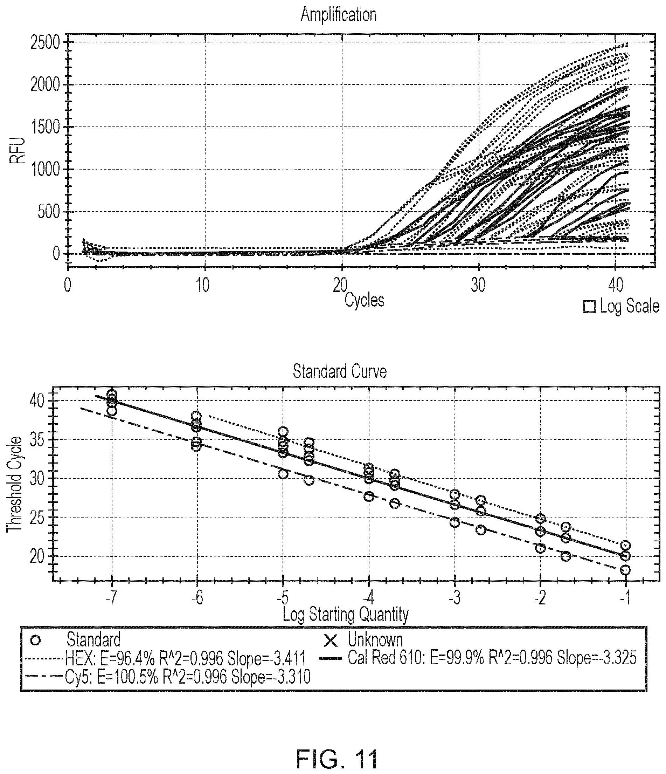

COL1A2 reaction did perform successfully in the individual reaction **(Figure 10)** and also in the multiplexed assay (ACTB, KRT19 and COL1A2) **(Figure 11).**

Conclusions

Reactions comprising ACTB, KRT19 and COL1A2 performed well in the multiplex assay. On the other hand reactions comprising COL11A1 did not perform well. For these reasons the combination of KRT19 and COL1A2, along with ACTB as the housekeeping gene were selected for use in the multiplex assays.

EXAMPLE 4

Validation of the biomarkers by multiplex RT-PCR

Materials and methods

Patients and samples

This study sample included a total of 60 cases, 47 cancer patients (CP) undergoing curative resection and 13 asymptomatic controls (AC) who were volunteer participants. Candidates with recurrence of neoplasia or other cancers were excluded from this research. The diagnoses, surgeries and sample collection were conducted by the University Hospital Complex of A Coruña (CHUAC). Samples were analyzed in the Hospital Pathology Department (UNE-EN ISO 9001-2015 certified). Clinical data such as gender, age, tumor histology or disease stage were obtained from the medical records. The study was done in compliance with the Declaration of Helsinki. Approval from the Clinical Research Ethics Committee, patients written informed consents custody and sample storage was managed by the Biobank of A Coruña. The demographic and clinical-pathological characteristics of the population are summarized in **Table 4.**

On the day of surgery, participants had a blood volume of 6-10 mL drawn into EDTA (K3) collection tubes (Vacuette, Greiner bio-one, Kremsmünster, Austria). The blood was centrifuged at room temperature at 2000 xg for 20 minutes. Tubes were processed during the first 4 hours and the plasma was stored at -80 °C. RNA was extracted from plasma samples using the ExoRNeasy Maxi Kit, (QIAGEN, Hilden, Germany) according to the supplier's instructions. In the elution step, the yield was increased by incubating the column with 14 uL of RNase-free water for 10 min.

**Table 4**

| Clinical and pathological features of patients included in this study (n = 60) | | | | |
|---|---|---|---|---|
| **Clinic pathological features** | **AC (n = 13)** | **CPs (n = 47)** | | |
| **Age median (range)** | | | 41.46 (26-58) | 70.36 (46-90) |
| **Gender** | | | | |
| | Female | | 9 (69.23%) | 24 (51.06%) |
| | Male | | 4 (30.77%) | 23 (48.94%) |
| **Curative resections** | | **-** | | 47 (100%) |

| Clinical and pathological features of patients with tumor process (n = 47) | | | | |
|---|---|---|---|---|
| **Colorectal cancer (CRC) (n = 31)** | | | **Prostate cancer (PC) (n = 3)** | |
| | Age mean (range) | 71.45 (40-90) | Age mean (range) | 71.66 (64-86) |
| | Histological Type | | Histological Type | |
| | Adenocarcinoma | 31 | Adenocarcinoma | 3 |
| | Clinical stage | | Gleason score | |
| | Tis/I/II | 20 | 7 (3+4)/ 7 (4+3) | 2 |
| | III | 11 | 8 (4+4) | 1 |

(continued)

| Renal cancer (RC) (n = 6) | | | Bladder cancer (BC) (n = 4) | | |
|---|---|---|---|---|---|
| Age mean (range) | 61.66 (44-79) | | Age mean (range) | 72.00 (58-86) | |
| Histological Type | | | Histological Type | | |
| CCRCC | 6 | | Urothelial | 4 | |
| Clinical stage | | | Clinical stage | | |
| FUHURMAN G 2/3 | 2 | | 0a | 1 | |
| I(1)/III(1)/IV(2) | 4 | | II | 3 | |

| Others (n = 3) | | | | |
|---|---|---|---|---|
| | Age | Sex | Histological Type | Clinical stage |
| Leiomyosarcoma | 74 | female | Renal leiomyosarcoma | III |
| Breast cancer | 74 | female | Ductal | I |
| Penile cancer | 66 | male | Epidermoid | II |

Measurement of RNA

Genes of interest were co-amplified using the QIAGEN® Multiplex PCR Kit (QIAGEN) into a CFX96 C1000 Thermal Cycler (Bio-Rad Laboratories, USA) in multiplex TaqMan assays. Compatible primer and probe sets that are specific for human gene targets were designed and synthesized by TIB Molbiol (Berlin, Germany).

A COL1A2 amplicon of 72 bp was amplified with PCR primers 5'-CCTGGACCAATGGGCTTA-3' (SEQ ID NO: 4) and 5'-GAAACCTTGAGGGCCTGG-3' (SEQ ID NO:5), KRT19 amplicon of 130 bp was amplified with PCR primers 5'-CAGCCACTACTACACGACCATC-3' (SEQ ID NO:1) and 5'-CAAACTTGGTTCGGAAGTCATC-3' (SEQ ID NO: 2) and ACTB amplicon of 99 bp was amplified with PCR primers 5'-CCACACTGTGCCCATCTACG -3' (SEQ ID NO: 7) and 5'-AGGATCTTCATGAGGTAGTCAGTCAG-3' (SEQ ID NO: 8).

A 5' hexachlorofluorescein (HEX) labeled probe, 3' BlackBerry™ Quencher 650 (BBQ-650™) and sequence 5'-TAGAGGCCCACCTGGTGCAGC-3' (SEQ ID NO: 6) were used for the COL1A2 gene, a 5' N-hydroxysuccinimide (LC610) labelled probe, 3' BBQ-650 and sequence 5'-CAGCCAGACGGGCATTGTCG-3' (SEQ ID NO 3) for the KRT19 gene and a 5' tetramethylindo(di)-carbocyanines (CY5) labelled probe, 3' BBQ-650 and sequence 5'-CAGGT CCAGACGCAGGA TGGC-3' (SEQ ID NO: 9) for the ACTB gene.

The reaction mixture (20 $\mu$L) included the following reagents: 7.8 $\mu$L H2O, 5 $\mu$L QuantiNova Multiplex PCR Master Mix (QIAGEN), 1 $\mu$L of each primer-probe mix (20x primer-probe mix includes 16 $\mu$M forward/reverse primers and 5 $\mu$M TaqMan probe), 0.2 $\mu$L QuantiNova Multiplex RT mix and 4 $\mu$L RNA template. Multiplex RT-qPCR conditions included a RT 50°C for 10 min, initial activation at 95°C for 2 min and 40 cycles of 95°C for 5 s, 62°C for 30 s.

The relative quantification of COL1A2 and KRT19 expression was performed using a comparative 2-$\Delta\Delta$Ct method, using ACTB gene expression to normalize the data. In all possible cases, two measurements were taken and then averaged. The cycle threshold number $\leq$39 with **a** sigmoidal curve was accepted as positive.

The nonparametric Mann-Whitney U-test was used for comparisons among groups. $\Delta$Ct and 2-$\Delta\Delta$Ct are represented as mean$\pm$SD and unpaired t-tests (two tailed) were used to test for differences between groups. A binary logistic regression model was used for analysing data using IBM SPSS® Statistics v27. For both biomarkers a cut-off value was established using Youden's J index (value combining highest sensitivity and specificity), through receiver operator characteristic (ROC) curve analysis, and areas under the curve (AUC) were calculated. When more than one value fulfilled this condition, the cut-off value allowing for higher sensitivity was chosen. The statistical significance was determined at $\alpha$-limit = 5% in all analysis.

Results

Ct values (amplification cycle number) for KRT19 and COL1A2 were normalized by subtraction of the Ct obtained for ACTB in the same sample, to obtain a relative Ct value, $\Delta$Ct. Both KRT19 and COL1A2 $\Delta$Ct values were higher in the cancer patient group than in the healthy donor group: median $\Delta$Ct for COL1A2 in AC was 10.88 (IQR 10.31-11.70) and for cancer patients was 7.64 (IQR 6.87-10.56), and the median $\Delta$Ct for KRT19 in AC was 10.95 (IQR 10.73-12.66) and for cancer patients was 9.12 (IQR 8.10-11.40), both showing statistical significance **(Figure 12A).**

Also, relative gene expression levels were evaluated (increase or decrease in expression) using the 2-$\Delta\Delta$Ct equation. **Figure 12B** represents mean $\pm$ SD of AC (1.40$\pm$1.25) and CP (9.68 $\pm$ 15.07) for COL1A2 and mean $\pm$ SD of AC (1.21$\pm$0.78) and CP (26.61 $\pm$ 45.46) for KRT19. Both groups were found to be significantly different when a non-parametric Mann Whitney U-test was performed, yielding a p-value <0.0001 for COL1A2 and p-value = 0.0031 for KRT19.

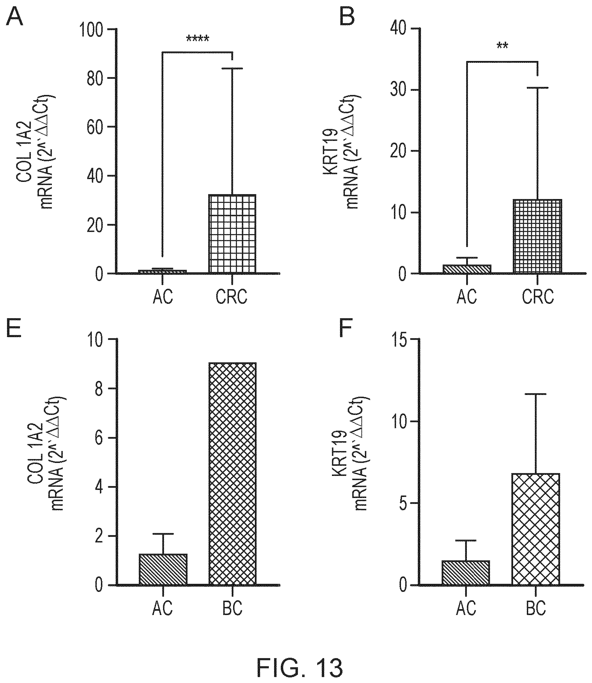

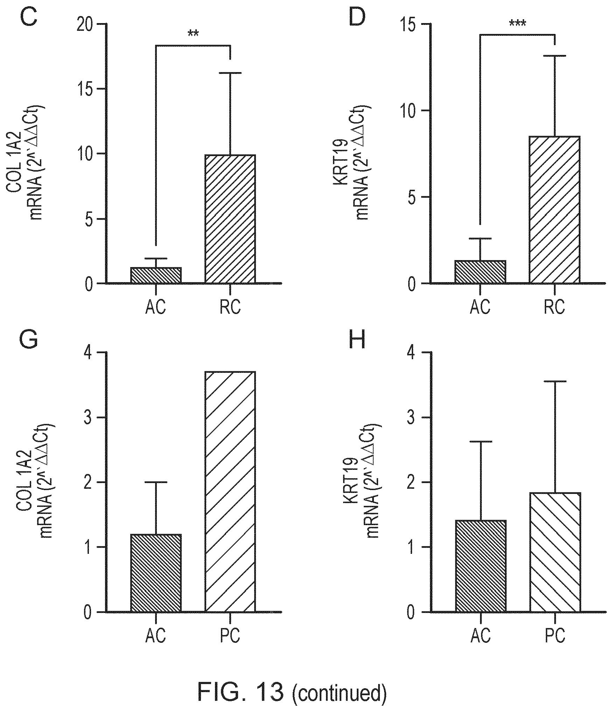

Differences in relative gene expression were also analyzed in the different cancer types individually **(Table 5** and **Figure 13).** In colorectal cancer (CRC) differences were significant for both COL1A2 (p-value<0.0001) and KRT19 (p-value = 0.0054). In renal cancer (RC) the differences were also significant for COL1A2 (p-value = 0.0088) and KRT19 (p-value = 0.0008). In bladder, prostate and other cancer types, the differences of individual biomarkers could not be evaluated, or were not significant (p values >0,1) due to the limited number of samples, even if a clear tendency to overexpression was observed in all of them, when comparing to the control group.

**Table 5**

| Groups | N | Amplification | | | mean $\pm$ SD | | p-values | |
|---|---|---|---|---|---|---|---|---|
| | | *COL1A2* | *KRT19* | *COL1A2 & KRT19* | *COL1A2* | *KRT19* | *COL1A2* | *KRT19* |
| **AC** | 13 | 12 (92.30%) | 13 (100%) | 12 (92.30%) | 1.21$\pm$0.78 | 1.40$\pm$1.25 | - | - |
| **CP** | 47 | 30 (63.83%) | 38 (80.85%) | 21 (44.68%) | 26.61$\pm$45.46 | 9.69$\pm$15.07 | 0.0001 | 0.0031 |
| **CRC** | 31 | 22 (70.96%) | 24 (77.42%) | 15 (48.39%) | 32.11$\pm$51.92 | 12.06$\pm$18.35 | 0.0001 | 0.0054 |
| **RC** | 6 | 3 (50%) | 5 (83.33%) | 1 (16.67%) | 9.82$\pm$6.50 | 8.50$\pm$4.75 | 0.0088 | 0.0008 |
| **BC** | 4 | 1 (25%) | 4 (100%) | 1 (25%) | nd | 6.74$\pm$4.89 | nd | 0.1567 |
| **PC** | 3 | 1 (33.33%) | 3 (100%) | 1 (33.33%) | nd | 1.84$\pm$1.71 | nd | 0.5054 |
| **Others** | 3 | 3 (100%) | 2 (66.67%) | 2 (66.67%) | 16.58$\pm$17.14 | nd | 0.0044 | nd |
| **Total** | 60 | 42 (70%) | 51 (85%) | 33 (55%) | nd | nd | nd | nd |

EXAMPLE 5

KRT19 and COL1A2 combination model

Method

A binary logistic regression model was used to analyze data using IBM SPSS® Statistics v27. For both biomarkers a cut-off value was established using Youden's J index (value combining highest sensitivity and specificity), through receiver operator characteristic (ROC) curve analysis, and areas under the curve (AUC) were calculated. When more than one value fulfilled this condition, the cut-off value allowing for higher sensitivity was chosen. The statistical significance was determined at $\alpha$-limit = 5% in all analysis.

Results

In order to design a diagnostic tool, we established an individual quantitative profile based on the level of biomarker expression. As not all patients express/amplify both markers, when one or both of the biomarkers (COL1A2 or KRT19) are not amplified, we considered that no mRNA was present and a value of 0 was assigned.

The algorithm that yielded the best classification of the patients was

$$p = \frac{1}{1 + e^{-(-1{,}396 + 0.550*KRT19 + 0.571*COL1A2)}}$$

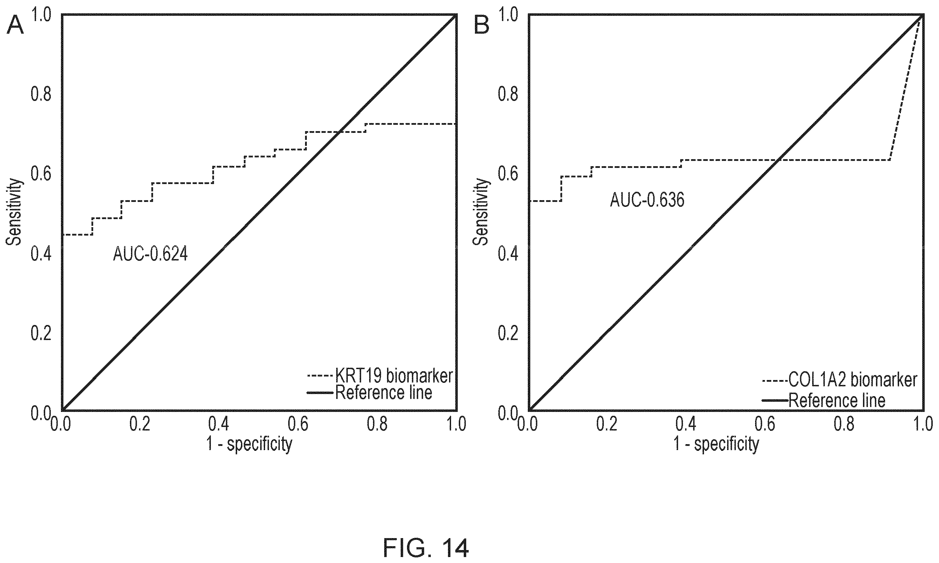

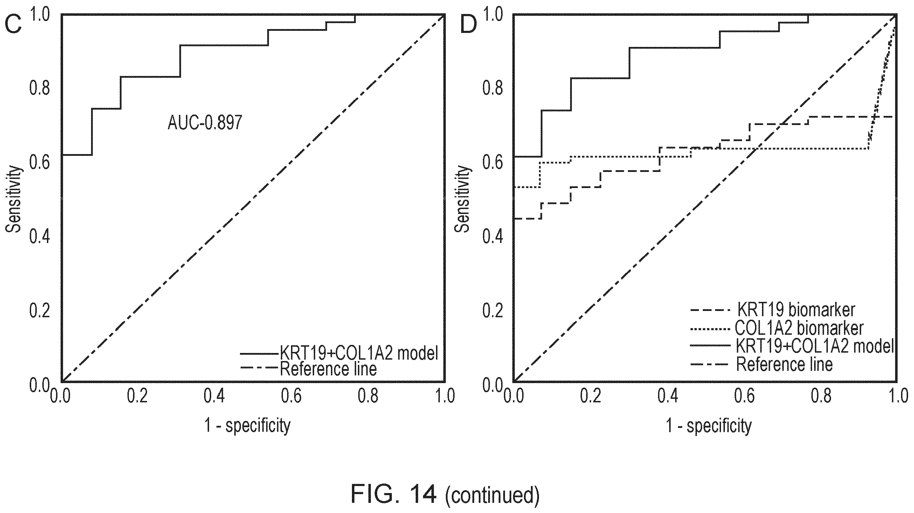

This quantitative expression profile is meant to be used as a patient classification tool, so a cut-off value was needed. For this purpose, ROC curve analysis was performed **(Figure 14).** Both biomarkers were used to identify the performing combination of the data set using logistic regression, which achieved AUC of 0.897 (0.815-0.979), as shown in Figure 14C. The AUC in the combined model (Figure 15C) was significantly higher than the individual biomarker model (Figures 14 A and B: AUC = 0.624 (0.490-0.757) for KRT19 and AUC = 0.636 (0.501-0.771) for COL1A2). The two biomarker model had an overall sensitivity of 0.830 and a specificity of 0.846 in the test, compared to individual marker's COL1A2 (S = 0.617, E = 0.845) and KRT19 (S = 0.617, E = 0.615) (Figures 14 A and B respectively)

Conclusions

The two-biomarker model yielded greater sensitivity and specificity than the individual biomarkers, performing better for patient classification. As the biomarker validation and the model construction were based on data of patients with resectable tumors, this diagnostic system may be useful for the detection of early tumors, and therefore could be a perfect screening tool that would allow detection of various types of cancers.

## REFERENCES CITED IN THE DESCRIPTION

Non-patent literature cited in the description

1. Esposito A.; Criscitiello C.; Locatelli M. et al. Liquid biopsies for solid tumors: understanding tumor heterogeneity and real time monitoring of early resistance to targeted therapies. Pharmacol. Ther. (2016); 157:120-124.

2. Gerlinger M.; Rowan A.J.; Horswell S. et al. Intratumor heterogeneity and branched evolution revealed by multiregion sequencing. N. Engl. J. Med. (2012); 366:883-892.

3. Feller S.M.; Lewitzky M. Hunting for the ultimate liquid cancer biopsy-let the TEP dance begin. Cell Commun. Signal. (2016); 14:24.

4. Izzotti A.; Carozzo S.; Pulliero A. et al. Extracellular microRNA in liquid biopsy: applicability in cancer diagnosis and prevention. Am. J. Cancer Res. (2016); 6:1461-1493.

5. Santarpia, M.; Liguori, A.; D'Aveni, A.; Karachaliou, N.; Gonzalez-Cao, M.; Daffinà, M.G.; Lazzari, C.; Altavilla, G.; Rosell, R. Liquid biopsy for lung cancer early detection. J. Thorac. Dis. 2018, 10, S882-S897; DOI:10.21037/jtd.2018.03.81.

6. Morán, T.; Felip, E.; Bosch-Barrera, J.; de Aguirre, I.; Ramirez, J.L.; Mesia, C.; Carcereny, E.; Roa, D.; Sais, E.; Garcia, Y.; et al. Monitoring EGFR-T790M mutation in serum/plasma for prediction of response to third-generation EGFR inhibitors in patients with lung cancer. Oncotarget 2018, 9, 27074-27086; DOI:10.18632/oncotarget.25478.

7. Huang, W.-L.; Chen, Y.-L.; Yang, S.-C.; Ho, C.-L.; Wei, F.; Wong, D.T.; Su, W.C.; Lin, C.-C. Liquid biopsy genotyping in lung cancer: ready for clinical utility? Oncotarget 2017, 8, 18590-18608; DOI:10.18632/oncotarget.14613.

8. Yu, D.; Li Y., Wang; M., Gu; J., Xu, W.; Cai, H.; Fang, X.; Zhang, X. Exosomes as a new frontier of cancer liquid biopsy. Mol Cancer. 2022 Feb 18;21(1):56. doi: 10.1186/s12943-022-01509-9.

9. Cai, X; Janku, F.; Zhan, Q.; Fan, J-B. Accessing genetic information with liquid biopsies. Trends Genet. 2015;31:564-75.

10. Yu, W.; Hurley, J.; Roberts, D.; Chakrabortty, S.; Enderle, D.; Noerholm, M.; et al. Exosome-based liquid biopsies in cancer: opportunities and challenges. Ann Oncol. 2021;32(4):466-77.

11. Möller, A.; Lobb, R.J. The evolving translational potential of small extracellular vesicles in cancer. Nat Rev Cancer. 2020 Dec;20(12):697-709. doi: 10.1038/s41568-020-00299-w.

12. Théry, C.; Witwer, K.W.; Aikawa, E.; Alcaraz, M.J.; Anderson, J.D.; Andriantsitohaina, R.; Antoniou, A.; Arab, T.; Archer, F.; Atkin-Smith, G.K; et al. Minimal information for studies of extracellular vesicles 2018 (MISEV2018): a position statement of the International Society for Extracellular Vesicles and update of the MISEV2014 guidelines. J Extracell Vesicles. 2018 Nov 23;7(1):1535750. doi: 10.1080/20013078.2018.1535750. eCollection 2018.

13. György, B.; Szabó, T.G.; Pásztói, M.; Pál, Z.; Misják, P.; Aradi, B.; László, V.; Pállinger, E.; Pap, E.; Kittel, A. Membrane vesicles, current state-of-the-art: emerging role of extracellular vesicles. Cell Mol Life Sci. 2011 Aug;68(16):2667-88. doi: 10.1007/s00018-011-0689-3.

14. Narang, P.; Shah, M.; Beljanski, V. Exosomal RNAs in diagnosis and therapies. Noncoding RNA Res. 2022 Jan 14;7(1):7-15. doi: 10.1016/j.ncrna.2022.01.001. eCollection 2022 Mar.

15. Becker, A.; Thakur, B.K.; Joshua Mitchell Weiss, J.M.; Kim, H.S.; Peinado, H.; Lyden, D. Extracellular Vesicles in Cancer: Cell-to-Cell Mediators of Metastasis. Cancer Cell. 2016 Dec 12;30(6):836-848. doi: 10.1016/j.ccell.2016.10.009.

16. Hoshino, A.; Costa-Silva, B.; Shen, T-L.; Rodrigues, G.; Hashimoto, A.; Mark, M.T.; Molina, H.; Kohsaka, S.; Di Giannatale, A.; Ceder, S.; et al. Tumour exosome integrins determine organotropic metastasis. Nature. 2015 Nov 19;527(7578):329-35. doi: 10.1038/nature15756. Epub 2015 Oct 28.

17. Qiao, F.; Pan, P.; Yan, J.; Sun, J.; Zong, Y.; Wu, Z.; Lu, X.; CheN, N.; Mi, R.; Ma, Y.; et al. Role of tumor-derived extracellular vesicles in cancer progression and their clinical applications. Int J Oncol. 2019 May;54(5):1525-1533. doi: 10.3892/ijo.2019.4745. Epub 2019 Mar 12.

18. Kok, V.C.; Yu, C-C. Cancer-Derived Exosomes: Their Role in Cancer Biology and Biomarker Development. Int J Nanomedicine. 2020 Oct 19;15:8019-8036. doi: 10.2147/IJN.S272378. eCollection 2020.

19. Xu, D.; Li, XF.; Zheng, S. et al. Quantitative real-time RT-PCR detection for CEA, CK20 and CK19 mRNA in peripheral blood of colorectal cancer patients. J Zhejiang Univ Sci B. (2006) 7(6):445-51.

20. Chu, PG, Weiss, LM. Keratin expression in human tissues and neoplasms. Histopathology 2002;40:403-39.

21. Masai K, Nakagawa K, Yoshida A, Sakurai H,. Watanabe S, Asamura H, Tsuta K. Cytokeratin 19 expression in primary thoracic tumors and lymph node metastasis. Lung Cancer. 2014; 86:3.

22. Xiao, Y.; Li, Y.; Yuan, Y.; Liu, B.; Pan, S.; Liu, Q.; Qi, X.; Zhou, H.; Dong, W.; Jia, L. The potential of exosomes derived from colorectal cancer as a biomarker. Clin. Chim. Acta 2019, 490, 186-193.

23. Bystricky, B.; Jurisova, S.; Karaba, M.; Minarik, G.; Benca, J.; Sedlácková, T.; Tothova, L.; Vlkova, B.; Cierna, Z.; Janega, P.; et al. Relationship Between Circulating Tumor Cells and Tissue Plasminogen Activator in Patients with Early Breast Cancer. Anticancer Res. 2017; 37(4):1787-1791.

24. Baghban, R.; Roshangar, L.; Jahanban-Esfahlan, R.; Seidi, K.; Ebrahimi-Kalan, A.; Jaymand, M.; Kolahian, S.; Javaheri, T.; Zare, P. Tumor microenvironment complexity and therapeutic implications at a glance. Cell Commun Signal. 2020 Apr 7;18:59.

25. Hanahan D, Coussens LM. Accessories to the crime: functions of cells recruited to the tumor microenvironment. Cancer Cell. 2012;21:309-22.

26. Korkaya, H.; Orsulic, S. Editorial: The tumor microenvironment: Recent advances and novel therapeutic approaches. Front Cell Dev Biol. 2020;8:586176.

27. Armstrong, T.; Packham, G.; Murphy, LB.; Bateman, AC.; Conti, JA.; Fine, DR.; et al. Type I collagen promotes the malignant phenotype of pancreatic ductal adenocarcinoma. Clinical Cancer Research: An Official Journal of the American Association for Cancer Research. 2004; 10:7427-7437.

28. Kehlet, SN.; Sanz-Pamplona, R.; Brix, S.; Leeming, DJ.; Karsdal, MA.; Moreno, V. Excessive collagen turnover products are released during colorectal cancer progression and elevated in serum from metastatic colorectal cancer patients. Scientific Reports. 2016; 6(1).

29. Bager, CL.; Willumsen, N.; Leeming, DJ.; Smith, V.; Karsdal, MA.; Dornan, D.; Bay-Jensen, AC. Collagen degradation products measured in serum can separate ovarian and breast cancer patients from healthy controls: A preliminary study. Cancer Biomarkers: Section A of Disease Markers. 2015; 15:783-788.

30. Identification of COL1A1 and COL1A2 as candidate prognostic factors in gastric cancer. Li J, et al., World J Surg Oncol. 2016 Nov 29;14(1):297

31. Search for new biomarkers of gastric cancer through serial analysis of gene expression and its clinical implications. Yasui W, et al., Cancer Sci. 2004 May;95(5):385-92

32. Positive association of collagen type I with non-muscle invasive bladder cancer progression. Brooks M, et al., Oncotarget. 2016 Dec 13;7(50):82609-82619

33. Expression and network analysis of YBX1 interactors for identification of new drug targets in lung adenocarcinoma. Murugesan SN, et al., J Genomics. 2018 Jun 26;6:103-112

34. CpG hypermethylation of collagen type I alpha 2 contributes to proliferation and migration activity of human bladder cancer. Mori K, et al., Int J Oncol. 2009 Jun;34(6):1593-602.

35. Pegtel, DM et al., Exosomes. Annu Rev Biochem . 2019 Jun 20;88:487-514.

36. One-step nucleic acid amplification for detecting lymph node metastasis of head and neck squamous cell carcinoma. Goda H, et al., Oral Oncol. 2012 Oct;48(10):958-963.

37. One-Step Nucleic Acid Amplification (OSNA): A fast molecular test based on CK19 mRNA concentration for assessment of lymph-nodes metastases in early stage endometrial cancer. Fanfani F, et al., PLoS One. 2018 Apr 26;13(4):e0195877.

38. An accurate and rapid detection of lymph node metastasis in non-small cell lung cancer patients based on one-step nucleic acid amplification assay. Inoue M, et al., Lung Cancer. 2012 Dec;78(3):212-8.

39. Lymph node metastases in papillary thyroid cancer detected by quantitative real-time polymerase chain reaction for thyroglobulin and cytokeratine-19. Kaczka K, et al., Pol J Pathol. 2013 Jun;64(2):90-5

40. A view on drug resistance in cancer. Vasan, N. et al., Nature. 2019 Nov; 575(7782): 299-309

41. Monitoring Therapy Efficiency in Cancer through Extracellular Vesicles. Stevic, I. et al., Cells. 2020 Jan; 9(1): 13

42. Brooks JD. Translational genomics: the challenge of developing cancer biomarkers. Genome Res. 2012;22(2):183-187.

## Claims

1. A method for screening, detecting or monitoring a tumor in a subject, the method comprising:

   a). measuring the expression levels of KRT19 and COL1A2 in a sample obtained from the subject; and
   b). combining the expression levels of KRT19 and COL1A2 to produce a score
   c). based on the score, determining whether the subject has a high likelihood or low likelihood of a tumor.

2. The method for screening or detecting a tumor according to claim 1 wherein:

   (a) the subject is at risk of having or developing a tumor;
   (b) the subject is at risk of having or developing a recurrence of a tumor; and/or
   (c) the subject is a human.

3. The method for screening, detecting or monitoring a tumor according to claim 1 or claim 2 wherein the sample obtained from the subject is a biological fluid, optionally wherein the biological fluid is blood, serum, plasma, nipple aspirate fluid or urine, further optionally wherein the expression levels of KRT19 and COL1A2 are measured from plasma obtained from the subject and/or wherein the expression levels of KRT19 and COL1A2 are measured from plasma derived exosomes obtained from the subject.

4. The method for screening, detecting or monitoring a tumor according to any one of the preceding claims wherein the tumor is cancerous, optionally wherein the cancer is a solid cancer, further optionally wherein the cancer is selected from bladder cancer, breast cancer, colorectal cancer, leiomyosarcoma, lung cancer, melanoma, ovarian cancer, pancreatic cancer, penile cancer, prostate cancer and renal cancer.

5. The method for screening, detecting or monitoring a tumor according to any one of the preceding claims wherein the method comprises measuring cDNA, RNA and/or protein expression levels of KRT19 and COL1A2, optionally wherein the RNA expression levels of KRT19 and COL1A2 are measured by RT-PCR.

6. The method for screening, detecting or monitoring a tumor according to any one of the preceding claims wherein combining the expression levels of KRT19 and COL1A2 to produce a score comprises using a logistic regression model.

7. The method for screening, detecting or monitoring a tumor according to any one of the preceding claims wherein combining the expression levels of KRT19 and COL1A2 to produce a score comprises:

   a). assigning a weight value to KRT19 expression to provide a weighted KRT19 expression level
   b). assigning a weight value to COL1A2 expression to provide a weighted COL1A2 expression level
   c). calculating the sum of the weighted KRT19 and COL1A2 expression levels
   d). using the sum obtained from step c) to produce a probabilistic score.

8. The method for screening, detecting or monitoring a tumor according to any one of the preceding claims wherein combining the expression levels of KRT19 and COL1A2 to produce a score comprises determining a probabilistic score using the following formula:

$$probabilistic\ score\ \ p = \frac{1}{1 + e^{-(-\beta_0 + \sum_{i=1}^{k} \beta_i x_i)}}$$

wherein $\beta_0$ is an intercept weight, $\beta$ is a vector of weights for each of k variables and x is a vector of variables associated with the sample, wherein the variables comprise KRT19 expression levels, COL1A2 expression levels and optionally variables derived therefrom.

9. The method for screening, detecting or monitoring a tumor according to any one of the preceding claims wherein determining based on the score whether the subject has a high likelihood or low likelihood of a tumor comprises comparing said score with a reference value, optionally wherein the reference value is obtained from a control sample or is a pre- determined threshold, further optionally wherein determining based on the score whether the subject has a high likelihood or low likelihood of a tumor comprises comparing said score with one or more predetermined thresholds, and determining that the subject has a high likelihood of a tumor if the score is above a first threshold, or determining that the subject has a low likelihood of a tumor if the score is below a second predetermined threshold, optionally wherein the first and second predetermined thresholds are the same.

10. A method for evaluating the efficacy of a treatment for cancer in a subject comprising

   a). measuring the expression levels of KRT19 and COL1A2 in a sample obtained from the subject at a first time point;
   b). combining the expression levels of KRT19 and COL1A2 obtained at the first time point to form a reference value;
   c). measuring the expression levels of KRT19 and COL1A2 in a sample obtained from the subject at a second time point;
   d). combining the expression levels of KRT19 and COL1A2 obtained at the second time point to produce a score,
   e). based on the score, determining whether there is a high likelihood the tumor has progressed, regressed or stayed in a steady state when compared to the reference value.

11. A method of providing a tool for characterizing a sample obtained from a subject, the method comprising:

a). providing KRT19 and COL1A2 expression data for a plurality of training samples associated with known tumor status, thereby forming a labelled training dataset;

b). training a machine learning model using the labelled training dataset to learn parameters for at least the variable KRT19 expression level and the variable COL1A2 expression level, wherein the trained machine learning model provides as output a probabilistic score for predicting whether the test sample came from a subject having a tumor or not having a tumor, based on input data comprising the KRT19 expression level and COL1A2 expression level of the test sample.

12. A computer-implemented method for screening, detecting or monitoring a tumor in a subject, the method comprising:

a). providing KRT19 and COL1A2 expression data from a subject;
b). combining the expression levels of KRT19 and COL1A2 to produce a score;
c). based on the score, classifying the subject as having a high likelihood or low likelihood of a tumor.

13. A system comprising:

a). a processor; and
b). a computer readable medium comprising instructions that, when executed by the processor, cause the processor to perform the method of claim 12.

14. A non-transitory computer readable medium or media comprising instructions that, when executed by at least one processor, cause the at least one processor to perform the method of claim 12.

15. Use of a kit for detecting a tumor and/or monitoring progression or regression of a tumor in a subject from a biological fluid sample obtained from the subject, the kit comprising reagents, and specific antibodies, primers or probes for detection and/or quantification of KRT19 and COL1A2 expression.

**Patentansprüche**

1. Verfahren zum Screenen, Detektieren oder Überwachen eines Tumors bei einem Individuum, wobei das Verfahren Folgendes umfasst:

a) Messen der Expressionsniveaus von KRT19 und COL1A2 in einer Probe, die von dem Individuum erhalten wurde; und
b) Kombinieren der Expressionsniveaus von KRT19 und COL1A2, um einen Wert zu erzeugen,
c) Bestimmen, ob das Individuum eine hohe Wahrscheinlichkeit oder niedrige Wahrscheinlichkeit für einen Tumor aufweist, basierend auf dem Wert.

2. Verfahren zum Screenen oder Detektieren eines Tumors nach Anspruch 1, wobei:

(a) das Individuum ein Risiko aufweist, einen Tumor zu haben oder zu entwickeln;
(b) das Individuum ein Risiko aufweist, ein Tumorrezidiv zu haben oder zu entwickeln; und/oder
(c) das Individuum ein Mensch ist.

3. Verfahren zum Screenen, Detektieren oder Überwachen eines Tumors nach Anspruch 1 oder Anspruch 2, wobei die vom Individuum erhaltene Probe eine biologische Flüssigkeit ist, wobei die biologische Flüssigkeit gegebenenfalls Blut, Serum, Plasma, Brustwarzenaspiratsflüssigkeit oder Urin ist, weiters gegebenenfalls wobei die Expressionsniveaus von KRT19 und COL1A2 in Plasma gemessen werden, das vom Individuum erhalten wurde, und/oder wobei die Expressionsniveaus von KRT19 und COL1A2 in Plasma gemessen werden, das von Exosomen stammt, die von dem Individuum erhalten wurden.

4. Verfahren zum Screenen, Detektieren oder Überwachen eines Tumors nach einem der vorangegangenen Ansprüche, wobei der Tumor kanzerös ist, wobei der Krebs gegebenenfalls ein solider Krebs ist, wobei der Krebs weiters gegebenenfalls aus Blasenkrebs, Brustkrebs, Kolorektalkrebs, Leiomyosarkomen, Lungenkrebs, Melanomen, Ovarialkrebs, Pankreaskrebs, Peniskrebs, Prostatakrebs und Nierenkrebs ausgewählt ist.

5. Verfahren zum Screenen, Detektieren oder Überwachen eines Tumors nach einem der vorangegangenen Ansprüche, wobei das Verfahren das Messen von cDNA, RNA und/oder Proteinexpressionsniveaus von KRT19 und COL1A2 umfasst, wobei die RNA-Expressionsniveaus von KRT19 und COL1A2 gegebenenfalls durch RT-PCR gemessen werden.

6. Verfahren zum Screenen, Detektieren oder Überwachen eines Tumors nach einem der vorangegangenen Ansprüche, wobei das Kombinieren der Expressionsniveaus von KRT19 und COL1A2 zum Erzeugen eines Werts die Verwendung eines logistischen Regressionsmodells umfasst.

7. Verfahren zum Screenen, Detektieren oder Überwachen eines Tumors nach einem der vorangegangenen Ansprüche, wobei das Kombinieren der Expressionsniveaus von KRT19 und COL1A2 zur Erzeugung eines Werts Folgendes umfasst:

   a) Zuordnen eines Gewichtswerts zur KRT19-Expression, um ein gewichtetes KRT19-Expressionsniveau bereitzustellen,
   b) Zuordnen eines Gewichtswerts zur COL1A2-Expression, um ein gewichtetes COL1A2-Expressionsniveau bereitzustellen,
   c) Berechnen der Summe aus dem gewichteten KRT19- und COL1A2-Expressionsni-veau,
   d) Verwenden der in Schritt c) erhaltenen Summe, um einen Wahrscheinlichkeitswert zu erzeugen.

8. Verfahren zum Screenen, Detektieren oder Überwachen eines Tumors nach einem der vorangegangenen Ansprüche, wobei das Kombinieren der Expressionsniveaus von KRT19 und COL1A2 zur Erzeugung eines Werts das Bestimmen eines Wahrscheinlichkeitswerts unter Verwendung der folgenden Formel umfasst:

$$Wahrscheinlichkeitswert\ p = \frac{1}{1 + e^{-(-\beta_0 + \Sigma_{i=1}^{k} \beta_i x_i)}}$$

worin $\beta_0$ ein Konstantenwert ist, $\beta$ ein Vektor der Gewichtungen für jede k-Variable ist und x ein Vektor von Variablen ist, die der Probe zugewiesen sind, wobei die Variablen KRT19-Expressionsniveaus, COL1A2-Expressionsniveaus und gegebenenfalls davon abgeleitete Variablen umfassen.

9. Verfahren zum Screenen, Detektieren oder Überwachen eines Tumors nach einem der vorangegangenen Ansprüche, wobei das Bestimmen, ob das Individuum eine hohe Wahrscheinlichkeit oder niedrige Wahrscheinlichkeit für einen Tumor aufweist, basierend auf dem Wert das Vergleichen des Werts mit einem Bezugswert umfasst, wobei der Bezugswert gegebenenfalls von einer Kontrollprobe erhalten wurde oder eine vorbestimmte Schwelle ist, wobei weiters das Bestimmen, ob das Individuum eine hohe Wahrscheinlichkeit oder niedrige Wahrscheinlichkeit für einen Tumor aufweist, basierend auf dem Wert gegebenenfalls das Vergleichen des Werts mit einer oder mehreren vorbestimmten Schwellen und das Bestimmen, dass das Individuum eine hohe Wahrscheinlichkeit für einen Tumor aufweist, wenn der Wert über einer ersten Schwelle liegt, oder das Bestimmen, dass das Individuum eine niedrige Wahrscheinlichkeit für einen Tumor aufweist, wenn der Wert unter einer zweiten Schwelle liegt, umfasst, wobei die erste und zweite vorbestimmte Schwelle gegebenenfalls gleich sind.

10. Verfahren zur Bewertung der Wirksamkeit einer Krebsbehandlung bei einem Individuum, das Folgendes umfasst:

    a) Messen der Expressionsniveaus von KRT19 und COL1A2 in einer Probe, die zu einem ersten Zeitpunkt vom Individuum erhalten wurde;
    b) Kombinieren der Expressionsniveaus von KRT19 und COL1A2, die zum ersten Zeitpunkt erhalten wurden, um einen Bezugswert zu bilden;
    c) Messen der Expressionsniveaus von KRT19 und COL1A2 in einer Probe, die zu einem zweiten Zeitpunkt vom Individuum erhalten wurde;
    d) Kombinieren der Expressionsniveaus von KRT19 und COL1A2, die zum zweiten Zeitpunkt erhalten wurden, um einen Wert zu erzeugen;
    e) Bestimmen, ob eine hohe Wahrscheinlichkeit besteht, dass der Tumor im Vergleich zum Bezugswert fortgeschritten ist, sich zurückgebildet hat oder in einem stabilen Zustand geblieben ist, basierend auf dem Wert.

11. Verfahren zur Bereitstellung eines Tools für die Charakterisierung einer Probe, die von einem Individuum erhalten wurde, wobei das Verfahren Folgendes umfasst:

a) Bereitstellen von KRT19- und COL1A2-Expressionsdaten für eine Vielzahl von Training-Proben, die einem bekannten Tumorzustand zugewiesen sind, wodurch ein markierter Training-Datensatz gebildet wird;
b) Trainieren eines Systems für maschinelles Lernen unter Verwendung des markierten Training-Datensatzes, damit dieses Parameter für zumindest die Variable KRT19-Expressionsniveau und die Variable COL1A2-Expressionsniveau lernt, wobei das trainierte Modell für maschinelles Lernen als Ausgabe einen Wahrscheinlichkeitswert bereitstellt, der basierend auf Eingabedaten, die das KRT19-Expressionsniveau und das COL1A2-Expressionsniveau der Testprobe umfassen, vorhersagt, ob die Testprobe von einem Individuum mit einem Tumor oder ohne Tumor stammt.

12. Computer-implementiertes Verfahren zum Screenen, Detektieren oder Überwachen eines Tumors bei einem Individuum, wobei das Verfahren Folgendes umfasst:

a) Bereitstellen von KRT19- und COL1A2-Expressionsdaten von einem Individuum;
b) Kombinieren der Expressionsniveaus von KRT19 und COL1A2, um einen Wert zu erzeugen;
c) Klassifizieren des Individuums als eine hohe Wahrscheinlichkeit oder eine niedrige Wahrscheinlichkeit für einen Tumor aufweisend basierend auf dem Wert.

13. System, das Folgendes umfasst:

a) einen Prozessor; und
b) ein computerlesbares Medium, das Anweisungen umfasst, die bei Ausführung durch den Prozessor bewirken, dass der Prozessor ein Verfahren nach Anspruch 12 ausführt.

14. Nichttransitorische(s) computerlesbare(s) Medium oder Medien, die Anweisungen umfassen, die bei Ausführung durch zumindest einen Prozessor bewirken, dass der zumindest eine Prozessor ein Verfahren nach Anspruch 12 ausführt.

15. Verwendung eines Sets zum Detektieren eines Tumors und/oder Überwachen des Fortschreitens oder der Regression eines Tumors bei einem Individuum anhand einer biologischen Flüssigkeitsprobe, die vom Individuum erhalten wurde, wobei das Set Reagenzien und spezifische Antikörper, Primer oder Sonden für die Detektion und/oder Quantifizierung von KRT19- und COL1A2-Expression umfasst.

**Revendications**

1. Procédé de dépistage, de détection ou de surveillance d'une tumeur chez un sujet, le procédé comprenant les étapes consistant à :

a). mesurer les niveaux d'expression de KRT19 et COL1A2 dans un échantillon obtenu auprès du sujet ; et
b). combiner les niveaux d'expression de KRT19 et COL1A2 pour produire un score
c). sur la base du score, déterminer si le sujet présente une probabilité élevée ou une faible probabilité de tumeur.

2. Procédé de dépistage ou de détection d'une tumeur selon la revendication 1, dans lequel :

(a) le sujet risque d'avoir ou de développer une tumeur ;
(b) le sujet risque d'avoir ou de développer une récidive d'une tumeur ; et/ou
(c) le sujet est un être humain.

3. Procédé de dépistage, de détection ou de surveillance d'une tumeur selon la revendication 1 ou la revendication 2, dans lequel l'échantillon obtenu auprès du sujet est un fluide biologique, facultativement dans lequel le fluide biologique est du sang, du sérum, du plasma, un fluide d'aspiration de mamelon ou de l'urine, facultativement en outre dans lequel les niveaux d'expression de KRT19 et COL1A2 sont mesurés à partir de plasma obtenu auprès du sujet et/ou dans lequel les niveaux d'expression de KRT19 et COL1A2 sont mesurés à partir d'exosomes dérivés de plasma obtenus auprès du sujet.

4. Procédé de dépistage, de détection ou de surveillance d'une tumeur selon l'une quelconque des revendications précédentes, dans lequel la tumeur est cancéreuse, facultativement dans lequel le cancer est un cancer solide, en outre facultativement dans lequel le cancer est choisi parmi le cancer de la vessie, le cancer du sein, le cancer colorectal, le léiomyosarcome, le cancer du poumon, le mélanome, le cancer de l'ovaire, le cancer du pancréas, le cancer du pénis, le cancer de la prostate et le cancer du rein.

5. Procédé de dépistage, de détection ou de surveillance d'une tumeur selon l'une quelconque des revendications précédentes, dans lequel le procédé comprend une mesure de niveaux d'expression d'ADNc, d'ARN et/ou de protéine de KRT19 et de COL1A2, facultativement dans lequel les niveaux d'expression d'ARN de KRT 19 et de COL1A2 sont mesurés par RT-PCR.

6. Procédé de dépistage, de détection ou de surveillance d'une tumeur selon l'une quelconque des revendications précédentes, dans lequel la combinaison des niveaux d'expression de KRT19 et COL1A2 pour produire un score comprend une utilisation d'un modèle de régression logistique.

7. Procédé de dépistage, de détection ou de surveillance d'une tumeur selon l'une quelconque des revendications précédentes, dans lequel la combinaison des niveaux d'expression de KRT19 et COL1A2 pour produire un score comprend les étapes consistant à :

   a). attribuer une valeur de pondération à l'expression de KRT19 pour fournir un niveau d'expression de KRT19 pondéré
   b). attribuer une valeur de pondération à l'expression de COL1A2 pour fournir un niveau d'expression de COL1A2 pondéré
   c). calculer la somme des niveaux d'expression de KRT19 et COL1A2 pondérés
   d). utiliser la somme obtenue à l'étape c) pour produire un score probabiliste.

8. Procédé de dépistage, de détection ou de surveillance d'une tumeur selon l'une quelconque des revendications précédentes, dans lequel la combinaison des niveaux d'expression de KRT19 et COL1A2 pour produire un score comprend la détermination d'un score probabiliste à l'aide de la formule suivante :

$$probabilistic\ score\ \ p = \frac{1}{1 + e^{-(-\beta_0 + \sum_{i=1}^{k} \beta_i x_i)}}$$

dans lequel $\beta$o est un poids d'interception, $\beta$ est un vecteur de poids pour chacune de k variables et x est un vecteur de variables associées à l'échantillon, dans lequel les variables comprennent des niveaux d'expression de KRT19, des niveaux d'expression de COL1A2 et facultativement des variables dérivées de ceux-ci.

9. Procédé de dépistage, de détection ou de surveillance d'une tumeur selon l'une quelconque des revendications précédentes, dans lequel la détermination, sur la base du score que le sujet présente une probabilité élevée ou une faible probabilité d'une tumeur comprend une comparaison dudit score avec une valeur de référence, facultativement dans lequel la valeur de référence est obtenue à partir d'un échantillon témoin ou est un seuil prédéterminé, en outre facultativement dans lequel la détermination, sur la base du score, que le sujet présente une probabilité élevée ou une faible probabilité d'une tumeur comprend une comparaison dudit score avec un ou plusieurs seuils prédéterminés, et une détermination que le sujet présente une probabilité élevée d'une tumeur si le score est supérieur à un premier seuil, ou une détermination que le sujet présente une faible probabilité d'une tumeur si le score est inférieur à un second seuil prédéterminé, facultativement dans lequel les premier et second seuils prédéterminés sont les mêmes.

10. Procédé pour évaluer l'efficacité d'un traitement du cancer chez un sujet, comprenant les étapes consistant à

   a). mesurer les niveaux d'expression de KRT19 et COL1A2 dans un échantillon obtenu auprès du sujet à un premier instant ;
   b). combiner les niveaux d'expression de KRT19 et COL1A2 obtenus au premier instant pour former une valeur de référence ;
   c). mesurer les niveaux d'expression de KRT19 et COL1A2 dans un échantillon obtenu auprès du sujet à un second instant ;
   d). combiner les niveaux d'expression de KRT19 et COL1A2 obtenus au second instant pour produire un score,
   e). sur la base du score, déterminer s'il existe une forte probabilité que la tumeur ait progressé, régressé ou soit restée dans un état stable par rapport à la valeur de référence.

11. Procédé de fourniture d'un outil pour caractériser un échantillon obtenu auprès d'un sujet, le procédé comprenant les étapes consistant à :

a). fournir des données d'expression de KRT19 et COL1A2 pour une pluralité d'échantillons d'entraînement associés à un statut de tumeur connu, en formant ainsi un ensemble de données d'entraînement étiqueté ;
b) entraîner un modèle d'apprentissage automatique en utilisant l'ensemble de données d'entraînement étiqueté pour apprendre des paramètres pour au moins le niveau d'expression de KRT19 variable et le niveau d'expression de COL1A2 variable, dans lequel le modèle d'apprentissage automatique formé fournit en sortie un score probabiliste pour prédire si l'échantillon de test provient d'un sujet ayant une tumeur ou n'ayant pas de tumeur, sur la base de données d'entrée comprenant le niveau d'expression de KRT19 et le niveau d'expression de COL1A2 de l'échantillon de test.

12. Procédé mis en œuvre par ordinateur pour dépister, détecter ou surveiller une tumeur chez un sujet, le procédé comprenant les étapes consistant à :

a). fournir des données d'expression de KRT19 et de COL1A2 à partir d'un sujet ;
b). combiner les niveaux d'expression de KRT19 et COL1A2 pour produire un score ;
c). sur la base du score, classer le sujet comme ayant une probabilité élevée ou faible probabilité d'une tumeur.

13. Système, comprenant :

a). un processeur ; et
b) un support lisible par ordinateur comprenant des instructions qui, lorsqu'elles sont exécutées par le processeur, amènent le processeur à exécuter le procédé selon la revendication 12.

14. Support(s) lisible(s) par ordinateur non transitoire(s) comprenant des instructions qui, lorsqu'elles sont exécutées par au moins un processeur, amènent le au moins un processeur à exécuter le procédé selon la revendication 12.

15. Utilisation d'un kit pour détecter une tumeur et/ou surveiller la progression ou la régression d'une tumeur chez un sujet à partir d'un échantillon de fluide biologique obtenu auprès du sujet, le kit comprenant des réactifs, et des anticorps, des amorces ou des sondes spécifiques pour une détection et/ou une quantification de l'expression de KRT19 et COL1A2.

| 1 μm | EHT = 20.00kV | Signal A = STEM | Brightness = 10.7 % |
| | WD = 3.2 mm | Mag = 25.00 K X | Contrast = 68.4 % |

| 1 μm | EHT = 20.00kV | Signal A = STEM | Brightness = 10.9 % |
| | WD = 3.2 mm | Mag = 25.00 K X | Contrast = 67.9 % |

FIG. 1

| 200 nm | EHT = 20.00kV | Signal A = SE2 | Brightness = 11.2 % |
| | WD = 3.2 mm | Mag = 100.00 K X | Contrast = 68.5 % |

| 200 nm | EHT = 20.00kV | Signal A = STEM | Brightness = 10.6 % |
| | WD = 3.2 mm | Mag = 100.00 K X | Contrast = 67.6 % |

FIG. 1 (continued)

FIG. 1 (continued)

FIG. 2

FIG. 2 (continued)

FIG. 3

FIG. 4

FIG. 5

KRT19    AUC: 1

CEA    AUC: 0,66

FIG. 5 (continued)

FIG. 6

FIG. 7

FIG. 8

FIG. 9

FIG. 10

FIG. 11

FIG. 12

FIG. 13

FIG. 13 (continued)

FIG. 14

FIG. 14 (continued)

## REFERENCES CITED IN THE DESCRIPTION

**Patent documents cited in the description**

- US 20050064455 A

- WO 2017153546 A

**Non-patent literature cited in the description**

- **CARÉN et al.** *BMC Cancer*, 2011
- **CSISZAR et al.** *J Peridontol*, 2007
- **DOWDY** ; **WEARDEN**. Statistics for Research. John Wiley & Sons, 1983
- **ESPOSITO A.** ; **CRISCITIELLO C.** ; **LOCATELLI M et al.** Liquid biopsies for solid tumors: understanding tumor heterogeneity and real time monitoring of early resistance to targeted therapies. *Pharmacol. Ther.*, 2016, vol. 157, 120-124
- **GERLINGER M** ; **ROWAN A.J** ; **HORSWELL S et al.** Intratumor heterogeneity and branched evolution revealed by multiregion sequencing. *N. Engl. J. Med*, 2012, vol. 366, 883-892
- **FELLER S.M** ; **LEWITZKY M**. Hunting for the ultimate liquid cancer biopsy-let the TEP dance begin. *Cell Commun. Signal.*, 2016, vol. 14, 24
- **IZZOTTI A** ; **CAROZZO S** ; **PULLIERO A. et al.** Extracellular microRNA in liquid biopsy: applicability in cancer diagnosis and prevention. *Am. J. Cancer Res.*, 2016, vol. 6, 1461-1493
- **SANTARPIA, M.** ; **LIGUORI, A.** ; **D'AVENI, A.** ; **KARACHALIOU, N** ; **GONZALEZ-CAO, M** ; **DAFFINÀ, M.G.** ; **LAZZARI, C** ; **ALTAVILLA, G** ; **ROSELL, R.** Liquid biopsy for lung cancer early detection. *J. Thorac. Dis.*, 2018, vol. 10, S882-S897
- **MORÁN, T.** ; **FELIP, E.** ; **BOSCH-BARRERA, J.** ; **DE AGUIRRE, I.** ; **RAMIREZ, J.L.** ; **MESIA, C** ; **CARCERENY, E.** ; **ROA, D** ; **SAIS, E.** ; **GARCIA, Y. et al.** Monitoring EGFR-T790M mutation in serum/plasma for prediction of response to third-generation EGFR inhibitors in patients with lung cancer. *Oncotarget*, 2018, vol. 9, 27074-27086
- **HUANG, W.-L** ; **CHEN, Y.-L** ; **YANG, S.-C** ; **HO, C.-L** ; **WEI, F.** ; **WONG, D.T** ; **SU, W.C** ; **LIN, C.-C**. Liquid biopsy genotyping in lung cancer: ready for clinical utility?. *Oncotarget*, 2017, vol. 8, 18590-18608
- **YU, D.** ; **LI Y., WANG** ; **M., GU** ; **J., XU, W.** ; **CAI, H.** ; **FANG, X** ; **ZHANG, X**. Exosomes as a new frontier of cancer liquid biopsy. *Mol Cancer*, 18 February 2022, vol. 21 (1), 56
- **CAI, X** ; **JANKU, F** ; **ZHAN, Q.** ; **FAN, J-B.** Accessing genetic information with liquid biopsies. *Trends Genet.*, 2015, vol. 31, 564-75

- **YU, W.** ; **HURLEY, J.** ; **ROBERTS, D** ; **CHAKRABORTTY, S** ; **ENDERLE, D.** ; **NOERHOLM, M. et al.** Exosome-based liquid biopsies in cancer: opportunities and challenges. *Ann Oncol.*, 2021, vol. 32 (4), 466-77
- **MÖLLER, A.** ; **LOBB, R.J**. The evolving translational potential of small extracellular vesicles in cancer. *Nat Rev Cancer.*, December 2020, vol. 20 (12), 697-709
- **THÉRY, C** ; **WITWER, K.W** ; **AIKAWA, E.** ; **ALCARAZ, M.J.** ; **ANDERSON, J.D.** ; **ANDRIANTSITOHAINA, R.** ; **ANTONIOU, A** ; **ARAB, T** ; **ARCHER, F.** ; **ATKIN-SMITH, G.K et al.** Minimal information for studies of extracellular vesicles 2018 (MISEV2018): a position statement of the International Society for Extracellular Vesicles and update of the MISEV2014 guidelines. *J Extracell Vesicles*, 23 November 2018, vol. 7 (1), 1535750
- **GYÖRGY, B.** ; **SZABÓ, T.G.** ; **PÁSZTÓI, M.** ; **PÁL, Z.** ; **MISJÁK, P** ; **ARADI, B** ; **LÁSZLÓ, V** ; **PÁLLINGER, E** ; **PAP, E.** ; **KITTEL, A**. Membrane vesicles, current state-of-the-art: emerging role of extracellular vesicles. *Cell Mol Life Sci.*, August 2011, vol. 68 (16), 2667-88
- **NARANG, P.** ; **SHAH, M.** ; **BELJANSKI, V**. Exosomal RNAs in diagnosis and therapies. *Noncoding RNA Res.*, 14 January 2022, vol. 7 (1), 7-15
- **BECKER, A.** ; **THAKUR, B.K** ; **JOSHUA MITCHELL WEISS, J.M** ; **KIM, H.S.** ; **PEINADO, H** ; **LYDEN, D.** Extracellular Vesicles in Cancer: Cell-to-Cell Mediators of Metastasis. *Cancer Cell*, 12 December 2016, vol. 30 (6), 836-848
- **HOSHINO, A** ; **COSTA-SILVA, B.** ; **SHEN, T-L.** ; **RODRIGUES, G** ; **HASHIMOTO, A** ; **MARK, M.T.** ; **MOLINA, H.** ; **KOHSAKA, S** ; **DI GIANNATALE, A.** ; **CEDER, S et al.** Tumour exosome integrins determine organotropic metastasis. *Nature*, 19 November 2015, vol. 527 (7578), 329-35
- **QIAO, F.** ; **PAN, P.** ; **YAN, J** ; **SUN, J.** ; **ZONG, Y** ; **WU, Z** ; **LU, X** ; **CHEN, N** ; **MI, R.** ; **MA, Y et al.** Role of tumor-derived extracellular vesicles in cancer progression and their clinical applications. *Int J Oncol.*, May 2019, vol. 54 (5), 1525-1533

- **KOK, V.C** ; **YU, C-C**. Cancer-Derived Exosomes: Their Role in Cancer Biology and Biomarker Development. *Int J Nanomedicine*, 19 October 2020, vol. 15, 8019-8036
- **XU, D.** ; **LI, XF** ; **ZHENG, S. et al.** Quantitative real-time RT-PCR detection for CEA, CK20 and CK19 mRNA in peripheral blood of colorectal cancer patients. *J Zhejiang Univ Sci B*, 2006, vol. 7 (6), 445-51
- **CHU, PG** ; **WEISS, LM**. Keratin expression in human tissues and neoplasms. *Histopathology*, 2002, vol. 40, 403-39
- **MASAI K** ; **NAKAGAWA K** ; **YOSHIDA A** ; **SAKURAI H** ; **WATANABE S** ; **ASAMURA H** ; **TSUTA K**. Cytokeratin 19 expression in primary thoracic tumors and lymph node metastasis. *Lung Cancer.*, 2014, vol. 86, 3
- **XIAO, Y** ; **LI, Y.** ; **YUAN, Y.** ; **LIU, B.** ; **PAN, S** ; **LIU, Q.** ; **QI, X** ; **ZHOU, H.** ; **DONG, W** ; **JIA, L.** The potential of exosomes derived from colorectal cancer as a biomarker. *Clin. Chim. Acta*, 2019, vol. 490, 186-193
- **BYSTRICKY, B.** ; **JURISOVA, S** ; **KARABA, M.** ; **MINARIK, G.** ; **BENCA, J.** ; **SEDLÁCKOVÁ, T** ; **TOTHOVA, L** ; **VLKOVA, B** ; **CIERNA, Z.** ; **JANEGA, P et al.** Relationship Between Circulating Tumor Cells and Tissue Plasminogen Activator in Patients with Early Breast Cancer. *Anticancer Res.*, 2017, vol. 37 (4), 1787-1791
- **BAGHBAN, R** ; **ROSHANGAR, L** ; **JAHANBAN-ESFAHLAN, R** ; **SEIDI, K** ; **EBRAHIMI-KALAN, A.** ; **JAYMAND, M.** ; **KOLAHIAN, S.** ; **JAVAHERI, T** ; **ZARE, P**. Tumor microenvironment complexity and therapeutic implications at a glance. *Cell Commun Signal.*, 07 April 2020, vol. 18, 59
- **HANAHAN D** ; **COUSSENS LM.** Accessories to the crime: functions of cells recruited to the tumor microenvironment. *Cancer Cell.*, 2012, vol. 21, 309-22
- **KORKAYA, H** ; **ORSULIC, S**. Editorial: The tumor microenvironment: Recent advances and novel therapeutic approaches. *Front Cell Dev Biol.*, 2020, vol. 8, 586176
- **ARMSTRONG, T.** ; **PACKHAM, G.** ; **MURPHY, LB** ; **BATEMAN, AC.** ; **CONTI, JA.** ; **FINE, DR et al.** Type I collagen promotes the malignant phenotype of pancreatic ductal adenocarcinoma. *Clinical Cancer Research: An Official Journal of the American Association for Cancer Research*, 2004, vol. 10, 7427-7437
- **KEHLET, SN** ; **SANZ-PAMPLONA, R.** ; **BRIX, S** ; **LEEMING, DJ** ; **KARSDAL, MA** ; **MORENO, V**. Excessive collagen turnover products are released during colorectal cancer progression and elevated in serum from metastatic colorectal cancer patients. *Scientific Reports.*, 2016, vol. 6 (1)
- **BAGER, CL.** ; **WILLUMSEN, N** ; **LEEMING, DJ** ; **SMITH, V.** ; **KARSDAL, MA** ; **DORNAN, D** ; **BAY-JENSEN, AC**. Collagen degradation products measured in serum can separate ovarian and breast cancer patients from healthy controls: A preliminary study. *Cancer Biomarkers: Section A of Disease Markers*, 2015, vol. 15, 783-788
- **LI J et al.** Identification of COL1A1 and COL1A2 as candidate prognostic factors in gastric cancer. *World J Surg Oncol.*, 29 November 2016, vol. 14 (1), 297
- **YASUI W et al.** Search for new biomarkers of gastric cancer through serial analysis of gene expression and its clinical implications. *Cancer Sci.*, May 2004, vol. 95 (5), 385-92
- **BROOKS M et al.** Positive association of collagen type I with non-muscle invasive bladder cancer progression. *Oncotarget*, 13 December 2016, vol. 7 (50), 82609-82619
- **MURUGESAN SN et al.** Expression and network analysis of YBX1 interactors for identification of new drug targets in lung adenocarcinoma. *J Genomics*, 26 June 2018, vol. 6, 103-112
- **MORI K et al.** CpG hypermethylation of collagen type I alpha 2 contributes to proliferation and migration activity of human bladder cancer. *Int J Oncol.*, June 2009, vol. 34 (6), 1593-602
- **PEGTEL, DM et al.** Exosomes. *Annu Rev Biochem*, 20 June 2019, vol. 88, 487-514
- **GODA H et al.** One-step nucleic acid amplification for detecting lymph node metastasis of head and neck squamous cell carcinoma. *Oral Oncol.*, October 2012, vol. 48 (10), 958-963
- **FANFANI F et al.** One-Step Nucleic Acid Amplification (OSNA): A fast molecular test based on CK19 mRNA concentration for assessment of lymph-nodes metastases in early stage endometrial cancer. *PLoS One.*, 26 April 2018, vol. 13 (4), e0195877
- **INOUE M et al.** An accurate and rapid detection of lymph node metastasis in non-small cell lung cancer patients based on one-step nucleic acid amplification assay. *Lung Cancer.*, December 2012, vol. 78 (3), 212-8
- **KACZKA K et al.** Lymph node metastases in papillary thyroid cancer detected by quantitative real-time polymerase chain reaction for thyroglobulin and cytokeratine-19. *Pol J Pathol.*, June 2013, vol. 64 (2), 90-5
- **VASAN, N. et al.** A view on drug resistance in cancer. *Nature*, November 2019, vol. 575 (7782), 299-309
- **STEVIC, I. et al.** Monitoring Therapy Efficiency in Cancer through Extracellular Vesicles. *Cells.*, January 2020, vol. 9 (1), 13
- **BROOKS JD**. Translational genomics: the challenge of developing cancer biomarkers. *Genome Res.*, 2012, vol. 22 (2), 183-187